# FASCICULE DE BREVET EUROPEEN

(11) **EP 0 877 754 B1**
(45) Date de publication et mention de la délivrance du brevet: **29.03.2006**
(21) Numéro de dépôt: 97901688.8
(22) Date de dépôt: 28.01.1997
(51) Int. Cl.: C07K 14/00

(54) **PROTEINES DE RESERVE DE PLANTES ENRICHIES EN ACIDES AMINES, NOTAMMENT GAMMA-ZEINE DE MAIS ENRICHIE EN LYSINE, PLANTES EXPRIMANT CES PROTEINES**
AN AMINOSAEURE ANGEREICHERTE SPEICHERPROTEINE, INSBESONDERE AN LYSIN ANGEREICHERTE MAIS-GAMMA-ZEINE, UND DIESE EXPRIMIERENDE PFLANZEN
AMINO ACID-ENRICHED PLANT PROTEIN RESERVES, PARTICULARLY LYSINE-ENRICHED MAIZE GAMMA-ZEIN, AND PLANTS EXPRESSING SUCH PROTEINS

(30) Priorité: 29.01.1996 FR 9601004
(43) Date de publication de la demande: 18.11.1998
(73) Titulaire: Biogemma, 75001 Paris (FR)
(72) Inventeur: LUDEVID, Dolorès, Centro de Investigacion y, E-08034 Barcelone (ES); TORRENT, Margarita, Centro de Investigacion y, E-08034 Barcelone (ES); ALVAREZ, Inaki, Centro de Investigacion y, E-08034 Barcelone (ES); PEREZ, Pascual, F-63110 Beaumont (FR)
(74) Mandataire: Flesselles, Bruno F.G.
(86) Numéro de dépôt international: PCT/FR1997/000167
(87) Numéro de publication internationale: WO 1997/028247

(56) Documents cités:
- EP-A- 0 208 418
- EP-A- 0 318 341
- WO-A-93/12230
- WO-A-93/15221
- WO-A-95/06128
- EMBL SEQUENCE DATABASE , REL.18, ACCESSION NO. M19784, 20-FEB-1989, XP002035349 FORNEY, J.D., ET AL.:
- CHEMICAL ABSTRACTS, vol. 104, no. 19, 1986 Columbus, Ohio, US; abstract no. 168751, SCHOTT, H., ET AL.: "Synthesis of fragments of the terminal inverted repeating units of macronuclear DNA from hypotrichous ciliates" XP002034741 & MAKROMOL. CHEM., vol. 187, no. 1, 1986, pages 81-104,
- EMBL SEQUENCE DATABASE ACCESSION NO.R40179, RELEASE 43, 8-05-1995., XP002034738 HILLIER, L., ET AL.: "yf70e09.s1 Homo sapiens cDNA clone 27461 3' similar to contains MER22 repetitive element"
- GCG-GENESEQ DATABASE ACCESSION NO. R28869, 23-MAR-1993, XP002034739 & WO 92 19248 A (UNIVERSITY OF WASHINGTON) 12 Novembre 1992
- THE JOURNAL OF BIOLOGICAL CHEMISTRY, vol. 270, no. 18, 5 Mai 1995, pages 10847-10854, XP002035350 QI, Z., ET AL.: "Reconstitution of neronal cdc2-like kinase from bacteria-expressed cdk5 and an active fragement of the brain-specific activator"
- PLANT CELL 6 (12). 1994. 1911-1922., XP002017847 GELI M I ET AL: "Two structural domains mediate two sequential events in gamma-Zein targeting: Protein endoplasmic reticulum retention and protein body formation." cité dans la demande
- PLANT MOL BIOL 16 (1). 1991. 117-128., XP002017848 OHTANI T ET AL: "NORMAL AND LYSINE-CONTAINING ZEINS ARE UNSTABLE IN TRANSGENIC TOBACCO SEEDS." cité dans la demande
- PLANT MOLECULAR BIOLOGY, vol. 34, Mai 1997, pages 139-149, XP002034740 TORRENT, M., ET AL.: "Lysine-rich modified gamma-zeins accumulate in protein bodies of transiently transformed maize endosperms"

### LISTE DE SEQUENCES

(1) INFORMATIONS GENERALES:
   (i) DEPOSANT:
      (A) NOM: BIOCEM
      (B) RUE: Campus Universitaire des Cezeaux - 24 avenue des Landais
      (C) VILLE: AUBIERE
      (E) PAYS: FRANCE
      (F) CODE POSTAL: 63170
   (ii) TITRE DE L' INVENTION: PROTEINES DE RESERVE DE PLANTES ENRICHIES EN ACIDES AMINES, NOTAMMENT GAMMA-ZEINE DE MAIS ENRICHIE EN LYSINE. PLANTES EXPRIMANT CES PROTEINES.
   (iii) NOMBRE DE SEQUENCES: 11
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (v) DONNEES DE LA DEMANDE ACTUELLE:
      NUMERO DE LA DEMANDE: PCT/FR 97/00167
(2) INFORMATIONS POUR LA SEQ ID NO: 1:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 1:
   CGATGAATTC AAACCAAAGC CAAAGCCGAA GCCAAAAGAA TTCA 44
(2) INFORMATIONS POUR LA SEQ ID NO: 2:
   (i) CARACTERISTIQUES DE LA SEQUENCE:
      (A) LONGUEUR: 46 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (xi) DESCRIPTION DE LA SEQUENCE: SEQ ID NO: 2:
   AGCTTGAATT CTTTTGGCTT CGGCTTTGGC TTTGGTTTGA ATTCAT 46
(2) INFORMATIONS POUR LA SEQ ID NO: 3:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 3:
   Ile Glu Phe Lys Pro Lys Pro Lys Pro Lys Pro Lys Glu Phe Leu Gln
   1               5                   10                  15
   Pro
(2) INFORMATIONS POUR LA SEQ ID NO: 4:
   (i) CARACTERISTIQUES DE LA SEQUENCE:
      (A) LONGUEUR: 28 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 4:
   Ile Glu Phe Lys Pro Lys Pro Lys Pro Lys Pro Lys Glu Phe Lys Pro
   1               5                   10                  15
   Lys Pro Lys Pro Lys Pro Lys Glu Phe Leu Gln Pro
               20                  25
(2) INFORMATIONS POUR LA SEQ ID NO: 5:
   (i) CARACTERISTIQUES DE LA SEQUENCE:
      (A) LONGUEUR: 20 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 5:
   Asp Gly Ile Asp Glu Phe Lys Pro Lys Pro Lys Pro Lys Pro Lys Glu
   1               5                   10                  15
   Phe Lys Leu Asp
               20
(2) INFORMATIONS POUR LA SEQ ID NO: 6:
   (i) CARACTERISTIQUES DE LA SEQUENCE:
      (A) LONGUEUR: 672 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:1..669
      (D) AUTRES INFORMATIONS:/note= "SEQUENCE CODANTE DE L'ADNc DE LA GAMMA-ZEINE ET SEQUENCE D'ACIDES AMINES CORRESPONDANTE"
   (xi) DESCRIPTION DE LA SEQUENCE: SEQ ID NO: 6:
   ATG AGG GTG TTG CTC GTT GCC CTC GCT CTC CTG GCT CTC GCT GCG AGC    48
   Met Arg Val Leu Leu Val Ala Leu Ala Leu Leu Ala Leu Ala Ala Ser
    1               5                  10                  15
   GCC ACC TCC ACG CAT ACA AGC GGC GGC TGC GGC TGC CAG CCA CCG CCG    96
   Ala Thr Ser Thr His Thr Ser Gly Gly Cys Gly Cys Gln Pro Pro Pro
               20                  25                  30
   CCG GTT CAT CTA CCG CCG CCG GTG CAT CTG CCA CCT CCG GTT CAC CTG   144
   Pro Val His Leu Pro Pro Pro Val His Leu Pro Pro Pro Val His Leu
           35                  40                  45
   CCA CCT CCG GTG CAT CTC CCA CCG CCG GTC CAC CTG CCG CCG CCG GTC   192
   Pro Pro Pro Val His Leu Pro Pro Pro Val His Leu Pro Pro Pro Val
       50                  55                  60
   CAC CTG CCA CCG CCG GTC CAT GTG CCG CCG CCG GTT CAT CTG CCG CCG   240
   His Leu Pro Pro Pro Val His Val Pro Pro Pro Val His Leu Pro Pro
   65                  70                  75                  80
   CCA CCA TGC CAC TAC CCT ACT CAA CCG CCC CGG CCT CAG CCT CAT CCC   288
   Pro Pro Cys His Tyr Pro Thr Gln Pro Pro Arg Pro Gln Pro His Pro
                   85                  90                  95
   CAG CCA CAC CCA TGC CCG TGC CAA CAG CCG CAT CCA AGC CCG TGC CAG   336
   Gln Pro His Pro Cys Pro Cys Gln Gln Pro His Pro Ser Pro Cys Gln
               100                 105                 110
   CTG CAG GGA ACC TGC GGC GTT GGC AGC ACC CCG ATC CTG GGC CAG TGC   384
   Leu Gln Gly Thr Cys Gly Val Gly Ser Thr Pro Ile Leu Gly Gln Cys
           115                 120                 125
   GTC GAG TTT CTG AGG CAT CAG TGC AGC CCG ACG GCG ACG CCC TAC TGC   432
   Val Glu Phe Leu Arg His Gln Cys Ser Pro Thr Ala Thr Pro Tyr Cys
       130                 135                 140
   TCG CCT CAG TGC CAG TCG TTG CGG CAG CAG TGT TGC CAG CAG CTC AGG   480
   Ser Pro Gln Cys Gln Ser Leu Arg Gln Gln Cys Cys Gln Gln Leu Arg
   145                 150                 155                 160
   CAG GTG GAG CCG CAG CAC CGG TAC CAG GCG ATC TTC GGC TTG GTC CTC   528
   Gln Val Glu Pro Gln His Arg Tyr Gln Ala Ile Phe Gly Leu Val Leu
                   165                 170                 175
   CAG TCC ATC CTG CAG CAG CAG CCG CAA AGC GGC CAG GTC GCG GGG CTG   576
   Gln Ser Ile Leu Gln Gln Gln Pro Gln Ser Gly Gln Val Ala Gly Leu
               180                 185                 190
   TTG GCG GCG CAG ATA GCG CAG CAA CTG ACG GCG ATG TGC GGC CTG CAG   624
   Leu Ala Ala Gln Ile Ala Gln Gln Leu Thr Ala Met Cys Gly Leu Gln
           195                 200                 205
   CAG CCG ACT CCA TGC CCC TAC GCT GCT GCC GGC GGT GTC CCC CAC       669
   Gln Pro Thr Pro Cys Pro Tyr Ala Ala Ala Gly Gly Val Pro His
       210                 215                 220
   TGA                                                               672
(2) INFORMATIONS POUR LA SEQ ID NO: 7:
   (i) CARACTERISTIQUES DE LA SEQUENCE:
      (A) LONGUEUR: 223 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 7:
   Met Arg Val Leu Leu Val Ala Leu Ala Leu Leu Ala Leu Ala Ala Ser
   1               5                   10                  15
   Ala Thr Ser Thr His Thr Ser Gly Gly Cys Gly Cys Gln Pro Pro Pro
               20                  25                  30
   Pro Val His Leu Pro Pro Pro Val His Leu Pro Pro Pro Val His Leu
           35                  40                  45
   Pro Pro Pro Val His Leu Pro Pro Pro Val His Leu Pro Pro Pro Val
       50                  55                  60
   His Leu Pro Pro Pro Val His Val Pro Pro Pro Val His Leu Pro Pro
   65                  70                  75                  80
   Pro Pro Cys His Tyr Pro Thr Gln Pro Pro Arg Pro Gln Pro His Pro
                   85                  90                  95
   Gln Pro His Pro Cys Pro Cys Gln Gln Pro His Pro Ser Pro Cys Gln
               100                 105                 110
   Leu Gln Gly Thr Cys Gly Val Gly Ser Thr Pro Ile Leu Gly Gln Cys
           115                 120                 125
   Val Glu Phe Leu Arg His Gln Cys Ser Pro Thr Ala Thr Pro Tyr Cys
       130                 135                 140
   Ser Pro Gln Cys Gln Ser Leu Arg Gln Gln Cys Cys Gln Gln Leu Arg
   145                 150                 155                 160
   Gln Val Glu Pro Gln His Arg Tyr Gln Ala Ile Phe Gly Leu Val Leu
                   165                 170                 175
   Gln Ser Ile Leu Gln Gln Gln Pro Gln Ser Gly Gln Val Ala Gly Leu
               180                 185                 190
   Leu Ala Ala Gln Ile Ala Gln Gln Leu Thr Ala Met Cys Gly Leu Gln
           195                 200                 205
   Gln Pro Thr Pro Cys Pro Tyr Ala Ala Ala Gly Gly Val Pro His
       210                 215                 220
(2) INFORMATIONS POUR LA SEQ ID NO: 8:
   (i) CARACTERISTIQUES DE LA SEQUENCE:
      (A) LONGUEUR: 693 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:1..690
      (D) AUTRES INFORMATIONS:/note= "SEQUENCE CODANTE DE L'ADNc DE LA H45GAMMA-Z ZEINE DE MAIS ET SEQUENCE D'ACIDES AMINES CORRESPONDANTE."
   (xi) DESCRIPTION DE LA SEQUENCE: SEQ ID NO: 8:
   ATG AGG GTG TTG CTC GTT GCC CTC GCT CTC CTG GCT CTC GCT GCG AGC      48
   Met Arg Val Leu Leu Val Ala Leu Ala Leu Leu Ala Leu Ala Ala Ser
       225                 230                 235
   GCC ACC TCC ACG CAT ACA AGC GGC GGC TGC GGC TGC CAG CCA CCG CCG      96
   Ala Thr Ser Thr His Thr Ser Gly Gly Cys Gly Cys Gln Pro Pro Pro
   240                 245                 250                 255
   CCG GTT CAT CTA CCG CCG CCG GTG CAT CTG CCA CCT CCG GTT CAC CTG     144
   Pro Val His Leu Pro Pro Pro Val His Leu Pro Pro Pro Val His Leu
                   260                 265                 270
   CCA CCT CCG GTG CAT CTC CCA CCG CCG GTC CAC CTG CCG CCG CCG GTC     192
   Pro Pro Pro Val His Leu Pro Pro Pro Val His Leu Pro Pro Pro Val
               275                 280                 285
   CAC CTG CCA CCG CCG GTC CAT GTG CCG CCG CCG GTT CAT CTG CCG CCG     240
   His Leu Pro Pro Pro Val His Val Pro Pro Pro Val His Leu Pro Pro
           290                 295                 300
   CCA CCA TGC CAC TAC CCT ACT CAA CCG CCC CGG ATC GAA TTC AAA CCA     288
   Pro Pro Cys His Tyr Pro Thr Gln Pro Pro Arg Ile Glu Phe Lys Pro
       305                 310                 315
   AAG CCA AAG CCG AAG CCA AAA GAA TTC AAA CCA AAG CCA AAG CCG AAG     336
   Lys Pro Lys Pro Lys Pro Lys Glu Phe Lys Pro Lys Pro Lys Pro Lys
   320                 325                 330                 335
   CCA AAA GAA TTC CTG CAG CCC CTG CAG GGA ACC TGC GGC GTT GGC AGC     384
   Pro Lys Glu Phe Leu Gln Pro Leu Gln Gly Thr Cys Gly Val Gly Ser
                   340                 345                 350
   ACC CCG ATC CTG GGC CAG TGC GTC GAG TTT CTG AGG CAT CAG TGC AGC     432
   Thr Pro Ile Leu Gly Gln Cys Val Glu Phe Leu Arg His Gln Cys Ser
               355                 360                 365
   CCG ACG GCG ACG CCC TAC TGC TCG CCT CAG TGC CAG TCG TTG CGG CAG     480
   Pro Thr Ala Thr Pro Tyr Cys Ser Pro Gln Cys Gln Ser Leu Arg Gln
           370                 375                 380
   CAG TGT TGC CAG CAG CTC AGG CAG GTG GAG CCG CAG CAC CGG TAC CAG        528
   Gln Cys Cys Gln Gln Leu Arg Gln Val Glu Pro Gln His Arg Tyr Gln
       385                 390                     395
   GCG ATC TTC GGC TTG GTC CTC CAG TCC ATC CTG CAG CAG CAG CCG CAA        576
   Ala Ile Phe Gly Leu Val Leu Gln Ser Ile Leu Gln Gln Gln Pro Gln
   400                 405                     410                 415
   AGC GGC CAG GTC GCG GGG CTG TTG GCG GCG CAG ATA GCG CAG CAA CTG        624
   Ser Gly Gln Val Ala Gly Leu Leu Ala Ala Gln Ile Ala Gln Gln Leu
                   420                 425                     430
   ACG GCG ATG TGC GGC CTG CAG CAG CCG ACT CCA TGC CCC TAC GCT GCT        672
   Thr Ala Met Cys Gly Leu Gln Gln Pro Thr Pro Cys Pro Tyr Ala Ala
               435                 440                     445
   GCC GGC GGT GTC CCC CAC TGA                                            693
   Ala Gly Gly Val Pro His
           450
(2) INFORMATIONS POUR LA SEQ ID NO: 9:
   (i) CARACTERISTIQUES DE LA SEQUENCE:
      (A) LONGUEUR: 230 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 9:
   Met Arg Val Leu Leu Val Ala Leu Ala Leu Leu Ala Leu Ala Ala Ser
    1               5                  10                  15
   Ala Thr Ser Thr His Thr Ser Gly Gly Cys Gly Cys Gln Pro Pro Pro
               20                  25                  30
   Pro Val His Leu Pro Pro Pro Val His Leu Pro Pro Pro Val His Leu
           35                  40                  45
   Pro Pro Pro Val His Leu Pro Pro Pro Val His Leu Pro Pro Pro Val
       50                  55                  60
   His Leu Pro Pro Pro Val His Val Pro Pro Pro Val His Leu Pro Pro
   65                  70                  75                  80
   Pro Pro Cys His Tyr Pro Thr Gln Pro Pro Arg Ile Glu Phe Lys Pro
                   85                  90                  95
   Lys Pro Lys Pro Lys Pro Lys Glu Phe Lys Pro Lys Pro Lys Pro Lys
               100                 105                 110
   Pro Lys Glu Phe Leu Gln Pro Leu Gln Gly Thr Cys Gly Val Gly Ser
           115                 120                 125
   Thr Pro Ile Leu Gly Gln Cys Val Glu Phe Leu Arg His Gln Cys Ser
       130                 135                 140
   Pro Thr Ala Thr Pro Tyr Cys Ser Pro Gln Cys Gln Ser Leu Arg Gln
   145                 150                 155                 160
   Gln Cys Cys Gln Gln Leu Arg Gln Val Glu Pro Gln His Arg Tyr Gln
                   165                 170                 175
   Ala Ile Phe Gly Leu Val Leu Gln Ser Ile Leu Gln Gln Gln Pro Gln
               180                 185                 190
   Ser Gly Gln Val Ala Gly Leu Leu Ala Ala Gln Ile Ala Gln Gln Leu
           195                 200                 205
   Thr Ala Met Cys Gly Leu Gln Gln Pro Thr Pro Cys Pro Tyr Ala Ala
       210                 215                 220
   Ala Gly Gly Val Pro His
   225                 230
(2) INFORMATIONS POUR LA SEQ ID NO: 10:
   (i) CARACTERISTIQUES DE LA SEQUENCE:
      (A) LONGUEUR: 723 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:1..720
      (D) AUTRES INFORMATIONS: /note= "SEQUENCE CODANTE DE L'ADNc de la P20GAMMAZ ZEINE DE MAIS ET SEQUENCE D'ACIDES AMINES CORRESPONDANTE."
   (xi) DESCRIPTION DE LA SEQUENCE: SEQ ID NO: 10:
   ATG AGG GTG TTG CTC GTT GCC CTC GCT CTC CTG GCT CTC GCT GCG AGC      48
   Met Arg Val Leu Leu Val Ala Leu Ala Leu Leu Ala Leu Ala Ala Ser
                   235                 240                 245
   GCC ACC TCC ACG CAT ACA AGC GGC GGC TGC GGC TGC CAG CCA CCG CCG      96
   Ala Thr Ser Thr His Thr Ser Gly Gly Cys Gly Cys Gln Pro Pro Pro
               250                 255                 260
   CCG GTT CAT CTA CCG CCG CCG GTG CAT CTG CCA CCT CCG GTT CAC CTG     144
   Pro Val His Leu Pro Pro Pro Val His Leu Pro Pro Pro Val His Leu
           265                 270                 275
   CCA CCT CCG GTG CAT CTC CCA CCG CCG GTC CAC CTG CCG CCG CCG GTC     192
   Pro Pro Pro Val His Leu Pro Pro Pro Val His Leu Pro Pro Pro Val
       280                 285                 290
   CAC CTG CCA CCG CCG GTC CAT GTG CCG CCG CCG GTT CAT CTG CCG CCG     240
   His Leu Pro Pro Pro Val His Val Pro Pro Pro Val His Leu Pro Pro
   295                 300                 305                 310
   CCA CCA TGC CAC TAC CCT ACT CAA CCG CCC CGG CCT CAG CCT CAT CCC     288
   Pro Pro Cys His Tyr Pro Thr Gln Pro Pro Arg Pro Gln Pro His Pro
                   315                 320                 325
   CAG CCA CAC CCA TGC CCG TGC CAA CAG CCG CAT CCA AGC CCG TGC CAG     336
   Gln Pro His Pro Cys Pro Cys Gln Gln Pro His Pro Ser Pro Cys Gln
               330                 335                 340
   ATC GAA TTC AAA CCA AAG CCA AAG CCG AAG CCA AAA GAA TTC CTG CAG     384
   Ile Glu Phe Lys Pro Lys Pro Lys Pro Lys Pro Lys Glu Phe Leu Gln
           345                 350                 355
   CCC CTG CAG GGA ACC TGC GGC GTT GGC AGC ACC CCG ATC CTG GGC CAG        432
   Pro Leu Gln Gly Thr Cys Gly Val Gly Ser Thr Pro Ile Leu Gly Gln
       360                 365                 370
   TGC GTC GAG TTT CTG AGG CAT CAG TGC AGC CCG ACG GCG ACG CCC TAC        480
   Cys Val Glu Phe Leu Arg His Gln Cys Ser Pro Thr Ala Thr Pro Tyr
   375                 380                 385                 390
   TGC TCG CCT CAG TGC CAG TCG TTG CGG CAG CAG TGT TGC CAG CAG CTC        528
   Cys Ser Pro Gln Cys Gln Ser Leu Arg Gln Gln Cys Cys Gln Gln Leu
                   395                 400                 405
   AGG CAG GTG GAG CCG CAG CAC CGG TAC CAG GCG ATC TTC GGC TTG GTC        576
   Arg Gln Val Glu Pro Gln His Arg Tyr Gln Ala Ile Phe Gly Leu Val
               410                 415                 420
   CTC CAG TCC ATC CTG CAG CAG CAG CCG CAA AGC GGC CAG GTC GCG GGG        624
   Leu Gln Ser Ile Leu Gln Gln Gln Pro Gln Ser Gly Gln Val Ala Gly
           425                 430                 435
   CTG TTG GCG GCG CAG ATA GCG CAG CAA CTG ACG GCG ATG TGC GGC CTG        672
   Leu Leu Ala Ala Gln Ile Ala Gln Gln Leu Thr Ala Met Cys Gly Leu
       440                 445                 450
   CAG CAG CCG ACT CCA TGC CCC TAC GCT GCT GCC GGC GGT GTC CCC CAC        720
   Gln Gln Pro Thr Pro Cys Pro Tyr Ala Ala Ala Gly Gly Val Pro His
   455                 460                 465                 470
   TGA                                                                    723
(2) INFORMATIONS POUR LA SEQ ID NO: 11:
   (i) CARACTERISTIQUES DE LA SEQUENCE:
      (A) LONGUEUR: 240 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 11:
   Met Arg Val Leu Leu Val Ala Leu Ala Leu Leu Ala Leu Ala Ala Ser
   1 5 10 15
   Ala Thr Ser Thr His Thr Ser Gly Gly Cys Gly Cys Gln Pro Pro Pro
   20 25 30
   Pro Val His Leu Pro Pro Pro Val His Leu Pro Pro Pro Val His Leu
   35 40 45
   Pro Pro Pro Val His Leu Pro Pro Pro Val His Leu Pro Pro Pro Val
   50 55 60
   His Leu Pro Pro Pro Val His Val Pro Pro Pro Val His Leu Pro Pro
   65 70 75 80
   Pro Pro Cys His Tyr Pro Thr Gln Pro Pro Arg Pro Gln Pro His Pro
   85 90 95
   Gln Pro His Pro Cys Pro Cys Gln Gln Pro His Pro Ser Pro Cys Gln
   100 105 110
   Ile Glu Phe Lys Pro Lys Pro Lys Pro Lys Pro Lys Glu Phe Leu Gln
   115 120 125
   Pro Leu Gln Gly Thr Cys Gly Val Gly Ser Thr Pro Ile Leu Gly Gln
   130 135 140
   Cys Val Glu Phe Leu Arg His Gln Cys Ser Pro Thr Ala Thr Pro Tyr
   145 150 155 160
   Cys Ser Pro Gln Cys Gln Ser Leu Arg Gln Gln Cys Cys Gln Gln Leu
   165 170 175
   Arg Gln Val Glu Pro Gln His Arg Tyr Gln Ala Ile Phe Gly Leu Val
   180 185 190
   Leu Gln Ser Ile Leu Gln Gln Gln Pro Gln Ser Gly Gln Val Ala Gly
   195 200 205
   Leu Leu Ala Ala Gln Ile Ala Gln Gln Leu Thr Ala Met Cys Gly Leu
   210 215 220
   Gln Gln Pro Thr Pro Cys Pro Tyr Ala Ala Ala Gly Gly Val Pro His
   225 230 235 240

## Description

La présente demande a pour objet de nouveaux moyens permettant la préparation de plantes exprimant des protéines de réserve enrichies en acides aminés déficitaires dans les protéines de réserve normales, et en particulier de protéines de réserves enrichies en lysine. L'invention a également pour objet les protéines de réserve ainsi modifiées, ainsi que des plantes exprimant ces protéines de réserve modifiées. Parmi ces protéines de réserve, l'invention concerne des protéines de réserve de la famille des zéines.

De nombreuses plantes, le cas échéant après transformation par des étapes physico-cnimiques, sont d'une importance économique majeure dans l'alimentation humaine ou animale, et le problème de l'amélioration de leur qualité nutritionnelle a déjà donné lieu à différents types de recherches. En particulier, pour remédier à l'insuffisante présence de certains acides aminés dans les protéines de réserve des plantes, on a mis au point des variétés sélectionnées présentant des qualités nutritionnelles supérieures, ou proposé différentes modifications faisant appel à l'utilisation des techniques du génie génétique afin de favoriser ou d'augmenter la production dans ces plantes notamment de certains acides aminés déficitaires, et néanmoins importants pour les qualités nutritionnelles de la plante. Ces acides aminés déficitaires sont par exemple la lysine, la méthionine.

Dans le cadre de la présente demande, les inventeurs se sont proposé d'apporter une solution originale au problème de l'amélioration des plantes, notamment de l'amélioration de leurs qualités nutritionnelles et ont à cet effet travaillé en premier lieu à partir d'une plante d'importance économique considérable, le maïs. Plus précisément, ils se sont intéressés aux protéines de réserve de l'endosperme des semences de maïs, protéines qui comprennent notamment les zéines et, en particulier, la γ-zéine.

Au cours du développement des semences de maïs, les cellules de l'endosperme synthétisent de grandes quantités de protéines de réserve, et notamment de α-, β-, et γ-zéines. Ces zéines sont accumulées dans les corps protéiques dérivés du réticulum endoplasmique (ER).

D'une façon générale, les zéines représentent un groupe complexe de protéines, réparties en plusieurs groupes, α-, β-, γ-, et δ-zéines (Larkins et al, 1989), codées par une famille multigénique (Hagen et Rubenstein, 1980, Gene 13, 239-249). Bien qu'elles présentent une structure variable, ces protéines ont des caractéristiques communes : la présence dans leur structure primaire de répétitions en tandem riches en résidus d'acides aminés de type Proline, la présence de nombreux résidus hydrophobes qui confèrent leur insolubilité à ces protéines dans un milieu aqueux, et l'absence de résidus lysine, acides aminés essentiels pour l'homme et les animaux monogastriques. L'absence de lysine dans toutes les protéines majoritaires (détectées en grande quantité dans l'endosperme) naturellement produites de la famille des zéines conduit à une composition en acides aminés déséquilibrée dans les semences de maïs.

Parmi ces protéines, la γ-zéine de maïs est une protéine ayant un poids moléculaire de 28 kDa, dont la séquence codante a été décrite sous la forme de l'ADNc par Prat et al (Nucleic Acids Research, vol. 13, n° 5, 1985, p. 1494-1504). La séquence complète du gène codant pour la γ-zéine, y compris les séquences non codantes en amont et en aval contenant les éléments de régulation de l'expression, a été décrite par Reina, M. et al (Nucleic Acids Research, vol. 18, n° 21, 1990, p 6426).

Jusqu'à présent, différentes approches ont été envisagées pour augmenter la teneur en lysine des protéines de la famille des zéines. A cet égard, des approches génétiques et moléculaires ont été mises en oeuvre. Par exemple, des mutants permettant l'obtention de mais riche en lysine, tels que le mutant opaque-2 (o2) et le mutant floury-2 (fl-2) (Mertz et al, 1964, Science 145, 279-280, Nelson et al, 1965, Science 150, 1469-1470) ont ainsi été proposés et des tentatives pour remédier aux effets néfastes de l'absence de certaines classes de zéines, notamment les α-zéines, sur les caractéristiques phénotypiques, par sélection de maïs contenant des gènes modificateurs o2 (Paez et al, 1969, Plant Sci. 9, 251-252, Geetha et al, 1991, Plant Cell 3,1207-1219) ont été faites.

Une autre approche a consisté à réaliser une action indirecte sur la production de lysine libre, en particulier dans les plantes dicotylédones. Cette technique a été mise en oeuvre en agissant sur la dérégulation des enzymes clé (DHTPS et AK) impliquées dans le cycle de la biosynthèse de la lysine via l'aspartate. Un accroissement sensible des niveaux de lysine libre a été obtenu dans les feuilles, mais pas dans les semences, dans des expériences de transformation de plantes de tabac avec des bactéries E. coli contenant les gènes dapA et des bactéries E. coli contenant le gêne lysc (Shaul et Galili, 1992, Plant J. 2, 203-209 et 1993, Plant Mol. Biol. 23, 759-768 ; Perl, A., Schaul O., Galili, G., 1992, Plant Molecular Biology, 19, p. 815-823). Récemment, les mêmes gènes dapA de Corynebacterium et lysC de E. coli ont été utilisés et exprimés sous le contrôle d'un promoteur spécifique des semences dans des plantes de soja. L'expression de ces deux enzymes dans le soja a conduit à une augmentation de cinq fois de la teneur en lysine dans les semences (Falco et al, 1995, BIO-Technology 13, 577-582).

D'autres auteurs (Wallace et al, 1988, Science 240, 662-664) ont tenté d'augmenter la lysine dans l'α-zéine (19 kDa) des semences de maïs en incorporant de façon ponctuelle des résidus lysine en différentes positions dans la molécule d'α-zéine. L'expression de ces constructions dans des oocytes de Xenopus a conduit à l'assemblage correct des zéines riches en lysine dans les vésicules analogues des corps protéiques. Cependant, l'α-zéine normale et la forme modifiée enrichie en lysine étaient dégradées lorsqu'elles étaient exprimées dans des semences de tabac (Othani et al, 1991, Plant Mol. Biol. 16:117).

On ne disposait donc pas à ce jour de connaissances quant aux moyens pouvant permettre l'expression d'une zéine enrichie en lysine dans les cellules la produisant naturellement dans le mais, c'est-à-dire dans les cellules de l'endosperme. A fortiori l'expression dans d'autres cellules végétales de zéines enrichies en lysine n'était pas maîtrisée.

L'un des buts de l'invention est donc de proposer des moyens permettant d'obtenir une zéine enrichie en lysine, en particulier une γ-zéine de maïs enrichie en lysine, cette protéine étant exprimée notamment dans les cellules des semences de maïs et en particulier dans les cellules de l'endosperme, ladite protéine modifiée étant en outre exprimée de façon telle que ses propriétés quant à la localisation et l'accumulation au niveau du réticulum endoplasmique et des corps protéiques dérivés soient préservées.

L'expression « enrichie en lysine » signifie, dans le contexte de la présente demande, que la protéine comporte un nombre augmenté de résidus lysine par rapport à la protéine naturelle dont elle est dérivée, par exemple par suite d'une modification de la séquence de nucléotides qui l'exprime.

L'invention propose aussi des moyens pour obtenir l'expression de protéines, de préférence de γ-zéines enrichies en lysine, dans des cellules végétales de différents tissus, par exemples des tissus de feuilles ou de racines, et ce le cas échéant dans des cellules végétales de plantes n'exprimant pas naturellement la protéine, en particulier la γ-zéine dont la production est recherchée.

Par ailleurs, selon un mode de réalisation particulier de l'invention, d'autres protéines de réserve peuvent être enrichies en lysine selon des modalités analogues, et plus particulièrement des protéines de réserve de la famille des zéines.

Les inventeurs se sont proposé dans un premier temps, pour réaliser l'invention, d'introduire dans le gène codant pour la γ-zéine ou pour d'autres protéines de réserve du maïs ou d'autres plantes, ou dans la séquence codante de ce gène, des séquences codant pour des polypeptides enrichis en lysine, afin d'obtenir la production de γ-zéines ou d'autres protéines enrichies en lysine et donc, de semences enrichies en lysine. Différents sites de la séquence codante du gène de la γ-zéine ont été identifiés comme sites permissifs, (appelés encore "sites neutres") pour préparer des séquences de nucléotides ainsi modifiées.

La présente demande propose donc des moyens pour transformer le gène codant pour la γ-zéine de maïs ou pour transformer toute séquence de nucléotides codant pour la γ-zéine et dérivée de ce gène, de façon à obtenir à partir de l'expression du gène modifié ou plus généralement de la séquence de nucléotides modifiée, une protéine enrichie en lysine; ces moyens comprennent en particulier des oligonucléotides synthétiques codant pour une séquence d'acides aminés comportant des résidus lysine.

L'invention a également pour objet des séquences de nucléotides recombinantes ou séquences chimères susceptibles de coder pour une γ-zéine enrichie en lysine.

Entrent également dans le cadre de l'invention des cellules hôtes transformées par de telles séquences, et en particulier des cellules végétales, par exemple des cellules permettant la régénération de plantes, ainsi que des plantes ou des parties de plantes (tissus, organes,...) contenant de telles cellules et produisant de façon stable des protéines de réserve modifiées, et en particulier des γ-zéines enrichies en lysine.

L'invention a également pour objet lesdites protéines modifiées, par exemple enrichies en lysine, ainsi que des anticorps dirigés contre ces protéines, et plus particulièrement des protéines de réserve de la famille des zéines, enrichies en lysine.

Un oligonucléotide approprié pour la réalisation de l'invention, utilisable pour la préparation de séquences de nucléotides recombinantes, est caractérisé en ce qu'il comprend au moins un enchaînement codant pour un polypeptide répondant à la formule (P-K)ₙ, dans laquelle:
- n est un entier supérieur ou égal à 2,
- P représente un résidu d'acide aminé Proline,
- K représente un résidu d'acide aminé Lysine,
- le signe "-" symbolise une liaison entre les deux résidus d'acides aminés et notamment une liaison de type peptidique, les n unités (P-K) étant liées entre elles également par de telles liaisons par exemple de type peptidique.

Un oligonucléotide selon l'invention est donc caractérisé selon un premier mode de réalisation, en ce qu'il comprend une séquence codant pour une suite de motifs répétés comportant deux acides aminés.

Les codons de l'oligonucléotide peuvent être identiques pour tous les résidus Proline et/ou pour tous les résidus lysine. Ils peuvent aussi être différents pour un même résidu d'acide aminé, la variation tenant compte de la dégénérescence du code génétique.

De préférence, cet oligonucléotide est formé par une séquence codant pour plus de 2 unités (P-K). De préférence n est inférieur ou égal à 30, en particulier inférieur à 20 et avantageusement n est égal à 4, 5, 6, 7, 8, 9 ou 10 ou 15.

Les "oligonucléotides" selon l'invention peuvent être synthétisés chimiquement par toute technique disponible.

Le terme "polypeptide" par référence à l'enchaînement (P-K)ₙ désigne, dans le cadre de la présente demande, une séquence d'acides aminés comportant plus de 2 résidus d'acides aminés et pouvant comporter jusqu'à 60 résidus d'acides aminés.

Selon une première variante de réalisation de l'invention, l'oligonucléotide comprend plusieurs enchaînements codant pour un polypeptide répondant à la formule (P-K)ₙ, identiques ou différents, associés en tandem.

Ces oligonucléotides sont soit des répétitions d'un même enchaînement, soit des associations d'enchaînement différents. Le nombre d'enchaînements associés est variable, pouvant par exemple être compris entre 2 et 10 enchaînements.

Selon une autre variante de réalisation de l'invention, l'oligonucléotide précédemment défini est caractérisé en ce qu'il comprend au moins un enchaînement codant pour un polypeptide répondant à la formule (P-K)ₙ, dans laquelle la séquence des n unités (P-K) est interrompue par un ou plusieurs résidus d'acides aminés différents des résidus P ou K.

De préférence, les acides aminés supplémentaires incorporés dans la séquence formée par les unités (P-K) sont choisis pour ne pas modifier l'organisation du polypeptide codé par l'oligonucléotide, où à tout le moins pour ne pas provoquer d'interaction avec des acides aminés d'une protéine dans laquelle ledit polypeptide serait incorporé, dans des conditions qui affecteraient la structure et/ou la fonction et/ou la localisation de cette protéine.

Ceci peut en particulier être le cas lorsque le nombre d'unités (P-K) est important ou que plusieurs enchaînements formés de séquences codant pour des motifs (P-K)ₙ sont associés en tandem et que la préparation de l'oligonucléotide correspondant nécessite que soient synthétisées plusieurs séquences nucléotides qui sont ensuite associées par exemple au moyen de séquences de liaison (linkers).

Selon un autre mode de réalisation de l'invention, l'oligonucléotide est tel que l'enchaînement codant pour le polypeptide comprenant les n unités (P-K) est complété à son extrémité 5' et/ou à son extrémité 3', par un ou plusieurs codons, codant par exemple pour au moins un résidu lysine à l'extrémité N-terminale du polypeptide.

A titre d'exemple, un oligonucléotide préféré selon l'invention est caractérisé en ce qu'il code pour un polypeptide répondant à la formule (P-K)ₙ, à la formule K-(P-K)₄, ou à la formule 2K (P-K)₄.

Selon un mode de réalisation particulier, la composition de cet oligonucléotide correspond à l'une des séquences décrites dans les pages qui suivent et identifiées par les désignations SEQ ID No:1 et SEQ ID No:2.

Les oligonucléotides précédemment décrits constituent des moyens de base pour réaliser des séquences de nucléotides recombinantes capables d'exprimer des protéines ou des polypeptides de réserve de plantes enrichis en lysine.

L'invention a donc aussi pour objet une séquence de nucléotides recombinante comprenant un enchaînement de nucléotides codant pour une protéine de réserve d'une plante, caractérisée en ce qu'elle comprend en outre un oligonucléotide selon l'invention, inséré en un site de l'enchaînement de nucléotides choisi de façon telle que :
- l'expression de la séquence de nucléotides dans une cellule végétale déterminée permet d'obtenir une protéine de réserve modifiée localisée de façon identique ou similaire dans cette cellule à la protéine de réserve normale qui serait exprimée dans la même cellule, dans les mêmes conditions par l'enchaînement de nucléotides normal codant correspondant, et/ou
- la protéine de réserve modifiée codée par la séquence de nucléotides est reconnue immunologiquement par des anticorps produits contre la protéine de réserve normale correspondante

En particulier, les anticorps précités sont constitués par un sérum polyclonal ou sont obtenus contre des épitopes de la protéine de réserve normale qui sont conservés dans la protéine de réserve modifiée.

Les cellules végétales auxquelles il est fait référence ci-dessus comprennent toute cellule végétale, quelle que soit son origine tissulaire ou sa nature. En particulier on s'intéresse dans le cadre de l'invention aux cellules des organes de réserve, mais aussi aux cellules de feuilles, tiges, tubercules...

Par l'expression "protéine de réserve" d'une plante, on entend dans le cadre de la présente demande une protéine synthétisée durant la maturation des semences et qui est utilisée pendant la phase de germination comme réserve principale de nutrition.

De façon générale, il s'agit d'un polypeptide susceptible d'être synthétisé dans un tissu de réserve quelle qu'en soit la localisation dans la plante ; les protéines de réserve mises en oeuvre dans le cadre de la présente demande sont en particulier celles produites dans les graines ou les semences des plantes de la famille des céréales, des crucifères ou des légumineuses, et sont par exemple les prolamines, les zéines.

Le choix du/des site(s) d'insertion de l'oligonucléotide dans l'enchaînement codant pour la protéine de réserve de plante est déterminé par le respect des conditions ci-dessus énoncées. Selon le cas, l'insertion peut avoir lieu dans un domaine répétitif (en termes de séquence d'acides aminés) de la protéine ou au niveau d'une extrémité C- ou N-terminale.

La condition donnée ci-dessus selon laquelle l'expression de la séquence de nucléotides recombinante de l'invention dans une cellule végétale permet d'obtenir une protéine de réserve modifiée, localisée identiquement ou similairement à la protéine de réserve normale qui serait exprimée dans les mêmes conditions dans la même cellule végétale, comporte par exemple pour les γ-zéines synthétisées, la possibilité d'être accumulées dans le réticulum endoplasmique des cellules végétales qui l'expriment, et en particulier dans les corps protéiques formés à partir de ce réticulum endoplasmique, lorsque la protéine est exprimée dans les cellules de l'endosperme.

Pour obtenir ce résultat au moyen des séquences de nucléotides recombinantes de l'invention, des systèmes d'expression adaptés à l'hôte cellulaire dans lequel est exprimée la séquence de nucléotides sélectionnée, et notamment les éléments de régulation, par exemple les promoteurs, sont choisis pour leur caractère fonctionnel dans le tissu contenant les cellules transformées. Des tests pour effectuer ce choix peuvent être réalisés en s'appuyant sur les différentes constructions décrites dans les exemples.

Pour vérifier que les propriétés immunologiques de la protéine de réserve modifiée exprimée par la séquence de nucléotides de l'invention n'ont pas été modifiées de façon conséquente, on a utilisé par exemple des antisérums tels que l'antisérum αG2, décrit précisément dans la partie expérimentale qui suit.

Selon un premier mode de réalisation de l'invention, la séquence de nucléotides recombinante est caractérisée en ce qu'elle est obtenue à partir d'un enchaînement codant de nucléotides qui conduit à l'expression d'une protéine de réserve naturellement pauvre en lysine.

De façon générale, cette séquence de nucléotides recombinante code pour une protéine de réserve modifiée dérivée d'une protéine de réserve naturellement produite par une plante utilisable en alimentation animale ou humaine.

Ainsi, les protéines de réserve dont on modifie la teneur en lysine dans le cadre de la présente invention sont avantageusement des protéines de réserve de plantes de la famille des céréales, des légumineuses ou des crucifères. Des protéines de réserve particulièrement importantes sont celles du maïs, en particulier les zéines, et plus spécialement la γ-zéine du maïs, dont on cherche à augmenter la teneur en lysine.

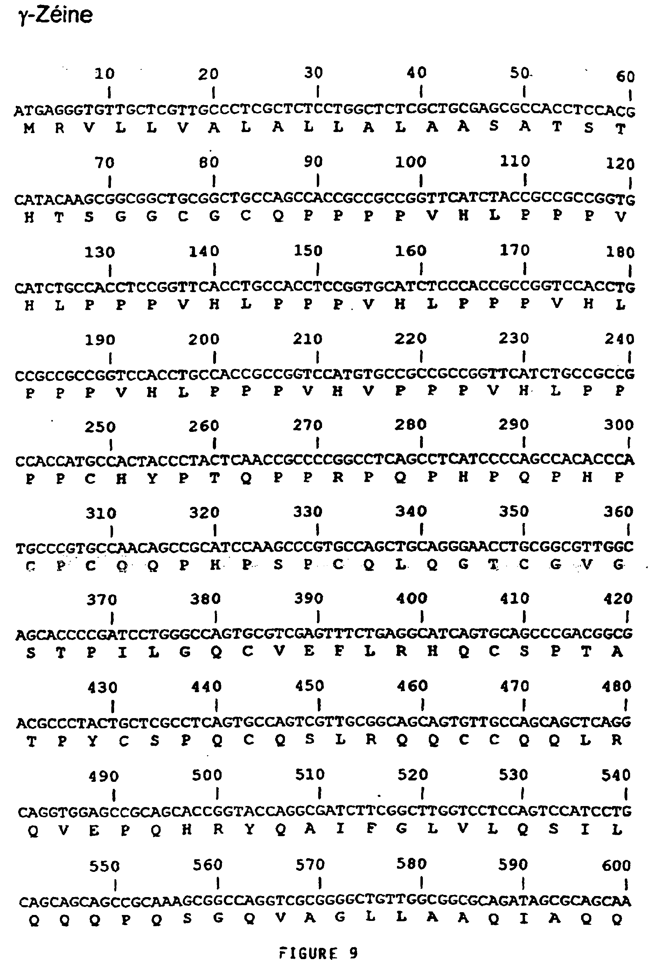

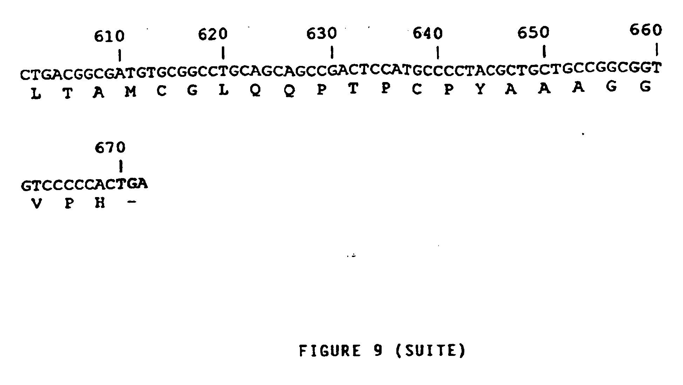

Une séquence de nucléotides recombinante particulière selon l'invention est caractérisée en ce que l'enchaînement codant de nucléotides codant pour la γ-zéine de maïs qu'elle contient, répond à la séquence présentée sur la Figure 9.

D'autres séquences de nucléotides recombinantes de l'invention sont caractérisées en ce que t'enchainement codant de nucléotides qu'elles comprennent, code pour une protéine de réserve d'une plante choisie parmi les suivantes : le soja, le tournesol, le tabac, le blé, l'avoine, la luzerne, le riz, le colza, le sorgo, Arabidopsis.

Selon un mode de réalisation préféré de l'invention, dans la séquence de nucléotides recombinante comprenant un enchaînement codant pour la γ-zéine de maïs, l'oligonucléotide de l'invention est inséré à la place de l'enchaînement codant pour le domaine Pro-X naturellement présent dans la séquence d'acides aminés de la γ-zéine du maïs ou à la suite de cet enchaînement. Le domaine Pro-X de la séquence d'acides aminés de la γ-zéine du maïs est constitué par les acides aminés situés entre les positions 70 et 91 de la séquence d'acides aminés représentée à la Figure 9, correspondant aux nucléotides 265 à 330 de la séquence représentée à la Figure 9.

De façon préférée, dans la séquence de nucléotides de l'invention, l'oligonucléotide à la place ou à la suite du domaine Pro-X présent entre les nucléotides 276 et 357 de la séquence représentée à la Figure 9.

Selon un autre mode de réalisation de l'invention, dans la séquence de nucléotides recombinante comprenant un enchaînement codant pour la γ-zéine de maïs, l'oligonucléotide de l'invention est inséré à la suite du domaine Pro-X conservé dans la séquence de la γ-zéine du maïs.

Selon une autre variante de réalisation, dans la séquence de nucléotides recombinante comprenant un enchaînement codant pour la γ-zéine de mais, l'oligonucléotide de l'invention est inséré dans le domaine Pro-X maintenu dans la séquence de la γ-zéine.

Les insertions dont il est question ci-dessus sont réalisables par les techniques disponibles, par exemple par recombinaison des séquences ayant subi une ou plusieurs étapes de digestion enzymatique.

Dans un mode de réalisation particulier de l'invention, une protéine de réserve choisie enrichie en un acide aminé déterminé est exprimée dans des cellules végétales hétérologues. En d'autres termes une protéine de réserve présente naturellement dans une plante donnée, est exprimée sous forme enrichie en acides aminés, dans une autre plante, ou dans une autre cellule que celle qui la produit naturellement

Outre l'enchaînement codant pour une protéine de réserve de plante et l'oligonucléotide de l'invention, les séquences de nucléotides recombinantes de l'invention peuvent également comprendre un promoteur d'expression et, par exemple, un promoteur choisi pour son caractère spécifique pour l'expression dans certaines parties ou dans certains tissus des plantes, ou au contraire un promoteur choisi pour son caractère constitutif. A titre d'exemple, lorsqu'ils sont spécifiques, les promoteurs peuvent être spécifiques des semences et/ou d'organes ou de tissus déterminés de plantes. Ils peuvent alternativement ou également être spécifiques d'une phase de croissance, par exemple d'un stade déterminé de la germination.

A l'inverse l'utilisation de promoteurs constitutifs permet l'expression constante et générale de la protéine de réserve, entraînant une compétition entre l'expression de la protéine de réserve native lorsqu'elle est présente et la protéine de réserve modifiée.

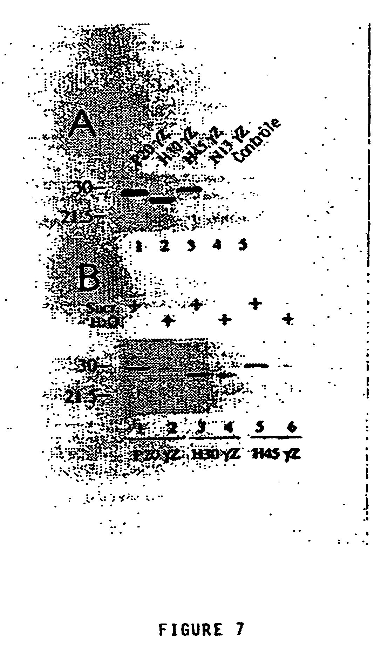

A titre d'exemple, des promoteurs avantageux pour la réalisation de l'invention sont le promoteur de la γ-zéine du maïs contenu dans la séquence de 1,7 kb se trouvant en amont de la séquence codante représentée à la Figure 7, le promoteur du virus de la mosaïque du chou-fleur, à savoir le promoteur CaMV35S (EP-B-0131623), le promoteur constitutif du gène d'actine 1 de riz (PCT/US 9100073) ou le promoteur semence spécifique "High Molecular weight gluthenine" du blé (Colot V. et al, 1987, EMBO Journal, vol. 6, p. 3559-3564).

Le cas échéant, ces promoteurs sont complétés par d'autres séquences de régulation, et en particulier des activateurs d'expression.

A titre d'exemple, d'autres promoteurs utilisables pour la réalisation de l'invention sont par exemple le promoteur du gène codant pour la protéine de réserve 2S des semences d'Arabidopsis thaliana, ou encore les promoteurs de la lectine de haricot ou de la β-phaséoline de haricot.

L'introduction supplémentaire d'activateurs de l'expression dans les séquences de régulation des séquences de nucléotides selon l'invention permet également d'augmenter le niveau de la transcription primaire de la séquence de nucléotides et, le cas échéant, d'augmenter la quantité de protéines de réserves modifiées produites. Des activateurs sont par exemple des introns de monocotylédones tels que l'intron 1 du gène d'actine de riz.

L'invention a également pour objet un vecteur de clonage et/ou d'expression, caractérisé en ce qu'il comprend, en un site non essentiel pour sa réplication, une séquence de nucléotides répondant à l'une des définitions précédemment données. Des vecteurs particuliers intéressants dans le cadre de la réalisation de l'invention sont par exemple les plasmides pP20γZ, pH30γZ ou pH45γZ Le plasmide pP20γZ a été déposé à la CNCM (Paris, FRANCE) le 31 Octobre 1995 sous le numéro I-1640. Le plasmide pH45γZ a été déposé à la CNCM le 31 Octobre 1995 sous le numéro I-1639.

Entre également dans le cadre de l'invention un polypeptide tel qu'exprimé par une séquence de nucléotides recombinante répondant aux définitions précédentes.

L'expression "polypeptide" n'introduit pas dans le cadre de la présente invention de limitation particulière quant au nombre d'acides aminés formant le polypeptide. Il peut donc s'agir de séquences comportant quelques acides aminés, habituellement désignées par l'expression "peptides", ou encore de séquences beaucoup plus longues telles que celles des protéines.

L'invention concerne à cet égard la γ-zéine de maïs modifiée riche en lysine, caractérisée en ce qu'elle est codée par une séquence de nucléotides recombinante décrite précédemment.

Selon un mode de réalisation préféré de l'invention, la γ-zéine de maïs modifiée enrichie en lysine est caractérisée en ce que sa séquence d'acides aminés est modifiée par au moins un polypeptide répondant à la formule (P-K)ₙ, dans laquelle :
- n est un entier supérieur ou égal à 2,
- P représente un résidu d'acide aminé Proline,
- K représente un résidu d'acide aminé Lysine,
- le signe "-" symbolise une liaison entre les deux résidus d'acides aminés et notamment une liaison de type peptidique, les n unités (P-K) étant liées par des liaisons notamment de type peptidique.

Selon une variante de réalisation de l'invention le polypeptide intégré dans la séquence d'acides aminés de la γ-zéine répond à la formule K-(P-K)ₙ.

Les polypeptides de l'invention répondant à l'une des formules (P-K)ₙ, K-(P-K)ₙ ou à des variantes sont substitués à une séquence naturellement présente dans la γ-zéine de maïs normale ou insérés avec délétion d'un ou plusieurs acides aminés de la séquence d'acides aminés de la γ-zéine de maïs normale, ou ajoutés dans la séquence d'acides aminés de la γ-zéine normale, le site d'insertion du polypeptide étant choisi de façon telle que :
- lorsque la γ-zéine modifiée riche en lysine est produite dans une cellule hôte, notamment une cellule végétale, elle est localisée de façon identique ou similaire dans cette cellule à la γ-zéine de maïs normale qui serait produite dans les mêmes conditions, dans la même cellule hôte et/ou,
- la γ-zéine de maïs modifiée est reconnue par des anticorps dirigés contre la γ-zéine normale de maïs.

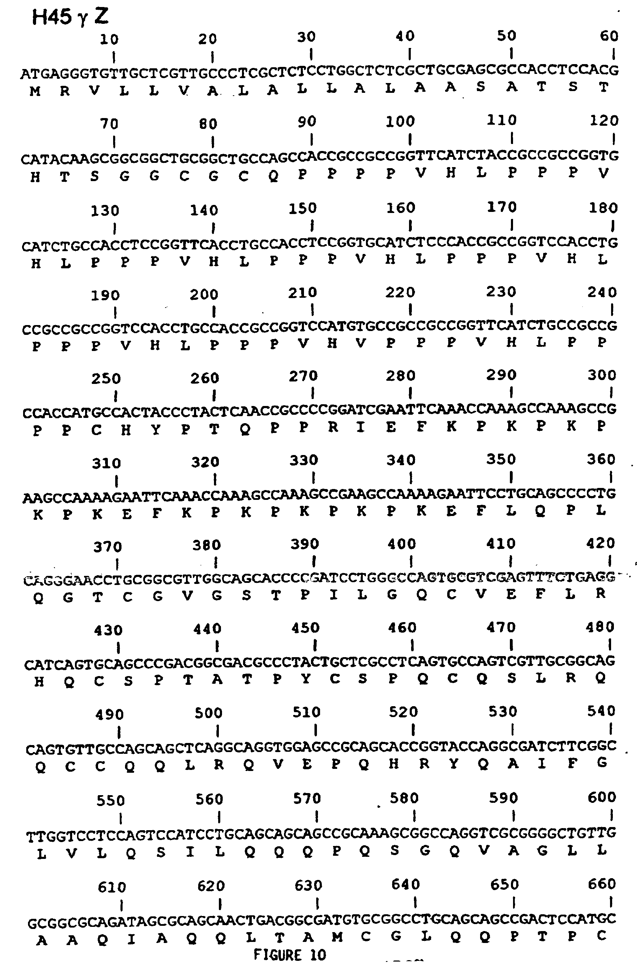

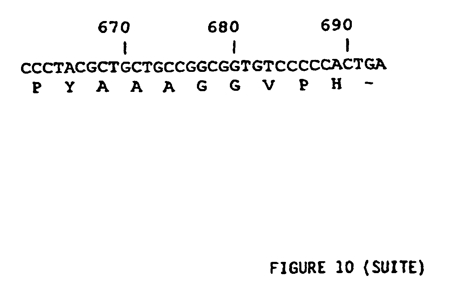

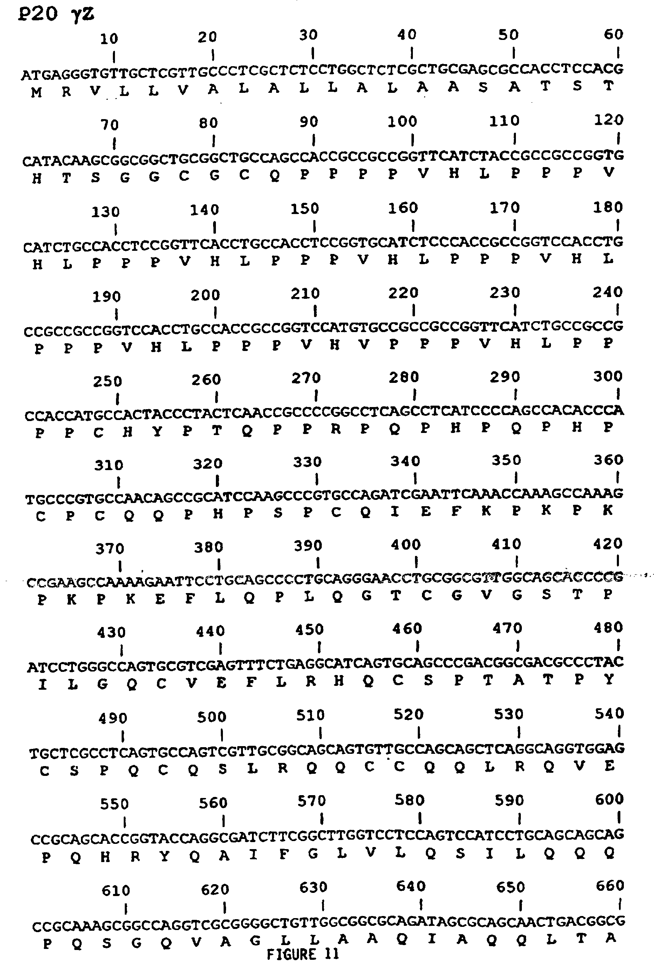

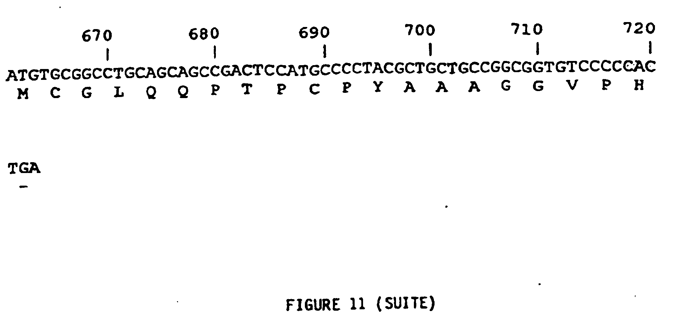

Les protéines P20γZ représentée à la figure 11 ou H30γZ ou H45γZ représentée à la figure 10, sont des exemples préférés de la réalisation de l'invention et représentent des γ-zéines de maïs modifiées enrichies en lysine.

L'invention a également pour objet une cellule hôte recombinante, caractérisée en ce qu'elle comprend une séquence de nucléotides décrite ci-dessus.

Des cellules hôtes intéressantes sont par exemple les cellules de bactéries, telles que celles de E. coli ou d'Agrobacteirum tumefaciens. De préférence, dans le cadre de l'invention, et pour l'expression stable de la protéine de réserve modifiée recherchée, on aura recours à des cellules hôtes d'origine végétale.

A titre d'exemple, ces cellules d'origine végétale sont des cellules de semences, de plante, et par exemple, à titre préféré, des cellules d'endosperme de semences de maïs.

La séquence de nucléotides de l'invention est de préférence introduite dans le génome de la cellule hôte de façon stable et dans des conditions telles que la protéine de réserve exprimée enrichie en acides aminés et en particulier en lysine est localisée comme le serait la protéine correspondante normale dans la même cellule hôte.

Des techniques variées sont disponibles pour transformer les cellules hôtes. A titre d'exemple, et en vue de transformer de façon stable ou de façon transitoire les cellules hôtes, on utilisera les techniques d'électroporation, de bombardement de microprojectiles portant de l'ADN, via canon à particules, via culture d'explants avec Agrobacterium tumefaciens, par pénétration de microfibres.

Outre les cellules d'endosperme de semences de maïs, des cellules de soja, de tournesol, de tabac, de blé, d'avoine, de luzerne, de riz, de colza, de sorgo ou d'Arabidopsis peuvent être mises en oeuvré pour exprimer les séquences de nucléotides de l'invention.

La présente demande concerne aussi les semences produisant un polypeptide tel que décrit ci-dessus et les plantes produisant ce même polypeptide. De préférence, ces plantes sont du maïs.

L'invention a aussi pour objet des semences obtenues à partir des plantes transformées exprimant le polypeptide de l'invention, en d'autres termes le polypeptide de réserve modifié enrichi en acides aminés déterminés.

Selon un mode de réalisation particulièrement intéressant de l'invention, les protéines γ-zéines modifiées enrichies en lysine, sont exprimées chez des mutants opaque 2 de maïs. Ces mutants o2 décrits par Emerson R.A. et al. (1935, Cornell Univ. Agric. Exp. Stn. Mem. 180) et caractérisés par Mertz E.T. et al (1964, Science 145 : 279-280) voient leur contenu en lysine fortement augmenté améliorant ainsi fortement les qualités nutritives du maïs (compensation de son bas niveau en cet acide aminé essentiel). Les maïs classiques ont un contenu en lysine d'environ 0,24% du produit brut (poids du grain total), les maïs opaque 2 avoisinent en revanche 0,5% en lysine. Cependant ils présentent des caractéristiques agronomiques insuffisantes car leur endosperme est beaucoup moins vitreux et se révèle très friable (phénotype "starchy"). Ceci a pour effet de les rendre extrèmement sensibles aux organismes pathogènes et aux étapes de traitements post recolte. Ce phénotype est en fait dû à la diminution importante de certaines protéines de réserve en particulier les alpha zéines. En fait Opaque 2 code pour un facteur de transcription nécessaire à l'expression de certains gènes zéines (Schmidt R. J. et al, 1990, Proc. Natl. Acad. Sci. USA 87, 46-50).

Des dérivés opaque 2 n'ayant plus les inconvénients précités ont été développés par amélioration génétique classique, il s'agit des maïs QPM (Quality Protein Maize). L'analyse génétique récente de ces maïs (Lopes M.A. et al, 1995, Theor. Appl. Genet. 19, 274-281) a montré que seulement 2 ou 3 loci se révèlent clefs dans ces modifications favorables. Des analyses génétiques et biochimiques plus poussées permettent de postuler qu'un des 3 loci responsables est le locus γ-zéine: les génotypes de maïs qui portent une duplication de ce gène situé dans la région centrométrique du chromosome 7, s'avèrent tous être des modificateurs d'opaque 2 (Lopes M.A. et al, 1995, Mol. Gen. Genet. 19 : 247 : 603-613).

La présente invention permet de préparer des maïs mutants opaque 2 à partir de maïs n'ayant qu'un gène de γ-zéine au niveau du chromosome 7, qui sont complémentés par l'addition d'une séquence recombinante codant pour une γ-zéine de mais enrichie en lysine. Outre le fait d'acquérir les propriétés de dureté similaire à un maïs opaque 2 non mutant, il a l'avantage d'augmenter significativement la teneur en lysine, dépassant ainsi celle des maïs QPM.

La présente invention permet d'obtenir des mutants opaque 2 de maïs modifiés, dans lesquels on a inséré une séquence de nucléotides recombinante codant pour une γ-zéine de maïs enrichie en lysine.

L'invention a aussi pour objet un procédé pour l'obtention de plantes ou de semences exprimant une protéine de réserve modifiée, caractérisé en ce qu'il comprend les étapes de :
a) transformation d'une cellule végétale, avec une séquence de nucléotides ou un vecteur ci-dessus décrits, dans des conditions permettant l'expression de façon stable et fonctionnelle de la protéine de réserve modifiée codée par la séquence de nucléotides;
b) régénération de plantes à partir de la cellule de plante transformée de l'étape a), pour obtenir des plantes exprimant la protéine de réserve modifiée,
c) le cas échéant, obtention des semences à partir des plantes modifiées obtenues à l'étape b).

Selon un mode de réalisation avantageux de l'invention, la plante transformée est le maïs et la protéine de réserve modifiée enrichie est la γ-zéine, enrichie en lysine.

L'invention concerne aussi les plantes obtenues par mise en oeuvre d'un tel procédé.

Afin d'évaluer la teneur en un acide aminé donné des plantes selon l'invention, il est possible d'utiliser un protocole, de dosage tel que celui mentionné dans Zarkadas et al, 1995, J. Agri. Food. Chem. Vol. 43: pages 84-93.

D'autres caractéristiques et avantages de l'invention apparaissent dans les exemples et dans les figures qui suivent.
**Figure 1**
   - Carte de restriction du plasmide pP20γZ
**Figure 2**
   - Carte de restriction du plasmide pH45γZ
**Figure 3**
   - Représentation schématique des protéines codées par les gènes modifiés et non modifiés de la γ-zéine : γ-zéine de type sauvage (γZ), γ-zéines riches en lysine (P20γZ, H30γZ, H45γZ et N13γZ) résultant de l'insertion des oligonucléotides codant pour des séquences riches en lysine. La séquence d'acides aminés des polypeptides insérés est indiquée en utilisant le code à une lettre de la représentation des acides aminés. Les abréviations suivantes sont utilisées :
      Term : terminal ;
      ProX DOMAIN : domaine linker proline-Xaa.

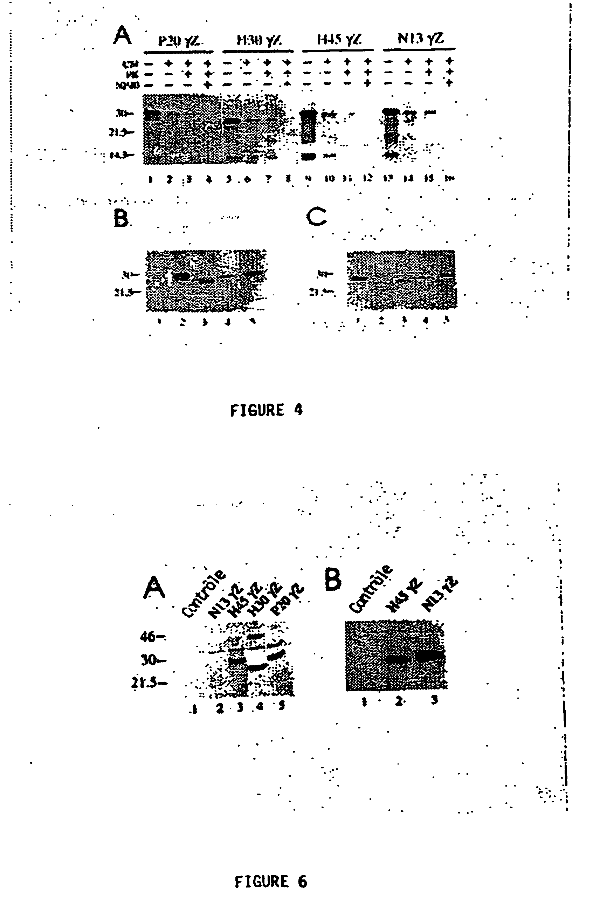

**Figure 4 -** Analyse in-vitro des γ-zéines riches en lysine. (A) traduction in-vitro et translocation des transcrits correspondant aux γ-zéines modifiées riches en lysine; lignes 1, 5, 9 et 13 : produits complets de traduction ; lignes 2, 6, 10 et 14 : produits complets de traduction après translocation dans les microsomes canins (CM); lignes 3, 7, 11 et 15: produits de translocation résistant à l'action de la protéinase K (PK) ; lignes 4, 8, 12 et 16 : totalité des produits de traduction après traitement avec la protéinase K en présence de 0,5% Nonidet P40(NP40). (B) Immunoprécipitation des produits de traduction in-vitro correspondant aux protéines γ-zéine et γ-zéine modifiées riches en lysine, en utilisant l'anti-sérum αPL. Ligne 1 : γ-zéine ; ligne 2 : P20γZ ; ligne 3 : H30γZ ; ligne 4 : H45γZ et ligne 5 : N13γZ. (C) Même légende que pour (B) mais en utilisant l'antisérum αG2. Les marqueurs de poids moléculaire (en kilodaltons) sont indiqués sur la gauche.

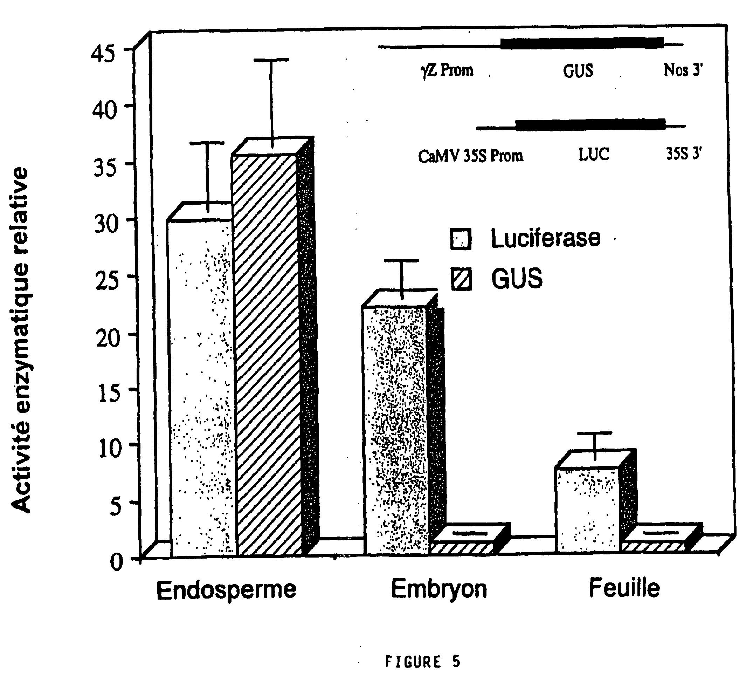

**Figure 5** - Activité tissu-spécifique du promoteur de la γ-zéine. Des endospermes de maïs, des embryons et des feuilles de maïs ont été co-transformés par bombardement de particules en utilisant les constructions représentées dans la figure (dans la partie à droite). Les activités relatives de la luciférase (LUC, colonnes en gris) et de la β-glucuronidase (GUS, colonnes hachurées) ont été exprimées sous la forme d'un multiplicateur des valeurs obtenues avec des projectiles nus ± la déviation standard des différents ratios.
**Figure 6 -** Expression des γ-zéines riches en lysine dans l'endosperme sub-aleuronique des cellules. (A) Immunoblot avec un antisérum αPL, de protéines extraites d'endospermes transformés par pN13γZ (ligne 2), pH45γZ (ligne 3), pH30γZ (ligne 4) et pP20γZ (ligne 5). Le témoin (ligne 1) correspond à des endospermes non transformés. Les marqueurs de poids moléculaire (en kilodaltons) sont indiqués à gauche. (B) expression des transcrits H45γZ et N13γZ dans des endospermes transformés de façon transitoire. Les cDNAs obtenus à partir des tissus transformés avec pH45γZ (ligne 2), pN13γZ (ligne 3) et le témoin (ligne 1) ont été amplifiés par PCR et analysés en utilisant un oligonucléotide synthétique codant pour une séquence riche en lysine à titre de sonde.
**Figure 7 -** Accumulation de γ-zéines riches en lysine dans les corps protéiques de l'endosperme. (A) Analyse en immunoblot en utilisant l'antisérum αPL, des corps protéiques isolés à partir des endospermes transformés avec pP20γZ (ligne 1), pH30γZ (ligne 2), pH45γZ (ligne 3), pN13γZ (ligne 4) et aucun ADN (ligne 5). (B) Analyse en immunoblot en utilisant l'antisérum αPL, des corps protéiques isolés à partir des endospermes transformés avec pP20γZ, pH30γZ et pH45γZ et digérés avec la protéinase K en présence d'un tampon isotonique (Sucr., lignes 1, 3 et 5) ou un tampon hypotonique (H₂O, lignes 2, 4 et 6). Les marqueurs de poids moléculaire (en kilodaltons) sont indiqués sur la gauche.

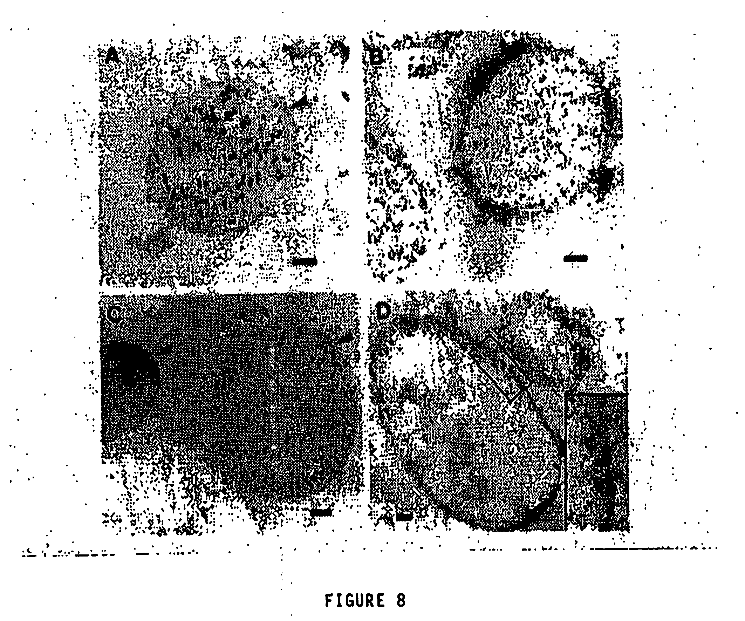

**Figure 8** - Co-localisation des protéines P20γZ avec les α- et les γ-zéines dans les corps protéiques de l'endosperme du maïs. Une analyse en immunocytochimie a été réalisée sur des sections ultrafines en utilisant des anticorps αPL (marqués avec des particules d'or de diamètre 15 nm) et des anticorps αZ et αG2 (marqués avec des particules d'or de 5 nm). (A) Corps protéiques de l'endosperme transformé avec pP20γZ, immunomarqués avec l'anticorps αPL. (B) Immunolocalisation de P20_{γ}Z (marqué avec des particules d'or de 15 nm) et de l'α-zéine (marquée avec les particules d'or de 5 nm) dans les corps protéiques isolés à partir des endospermes transformés avec pP20γZ. (C) et (D) Immunolocalisation de P20γZ (marqué avec les particules d'or de 15 nm) et des γ-zéines (marquées avec les particules d'or de 5 nm) dans des corps protéiques isolés à partir des endospermes transformés avec pP20γZ. Les flèches indiquent les sections tangentielles des corps protéiques.
**Figure 9** - Séquence codante de l'ADNc la γ-zéine de maïs et la séquence d'acides aminés correspondante.
**Figure 10 -** Séquence codante de l'ADNc de la H45γZ zéine de maïs et la séquence d'acides aminés correspondante.
   La séquence riche en lysine (28 acides aminés) est introduite entre les résidus d'acides aminés 92 et 119 de la séquence représentée à la Figure 10.
**Figure 11** - Séquence codante de l'ADNc de la P20γZ zéine de maïs et la séquence d'acides aminés correspondante.
   La séquence riche en lysine (14 acides aminés) est introduite entre les résidus d'acides aminés 92 et 119 de la séquence représentée à la Figure 11.

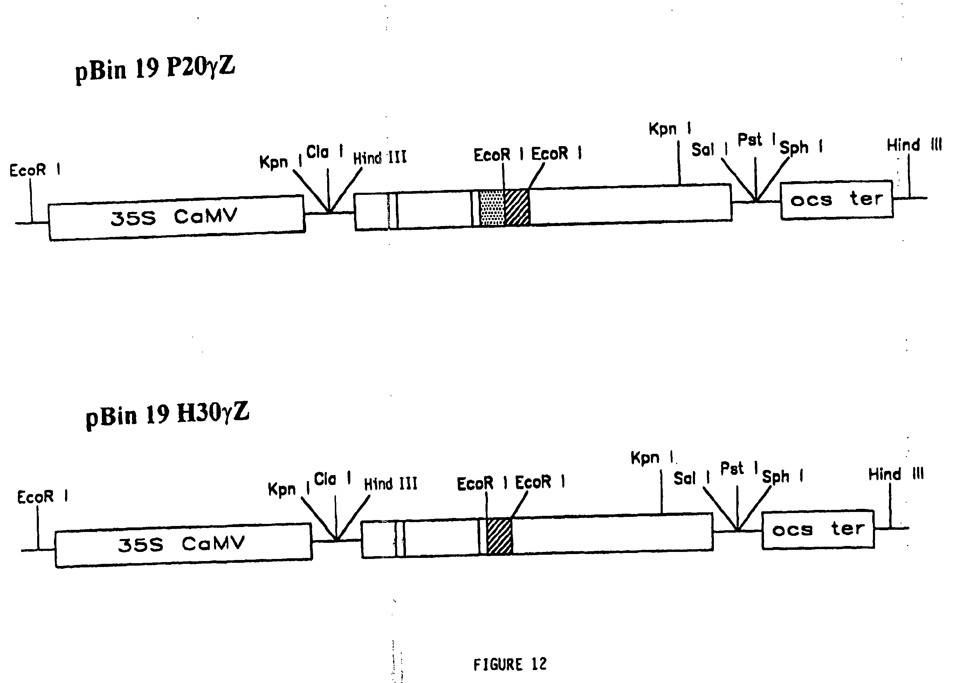

**Figure 12** - Cartes de restriction des plasmides pBin 19P20γZ et pBin 19H30γZ.

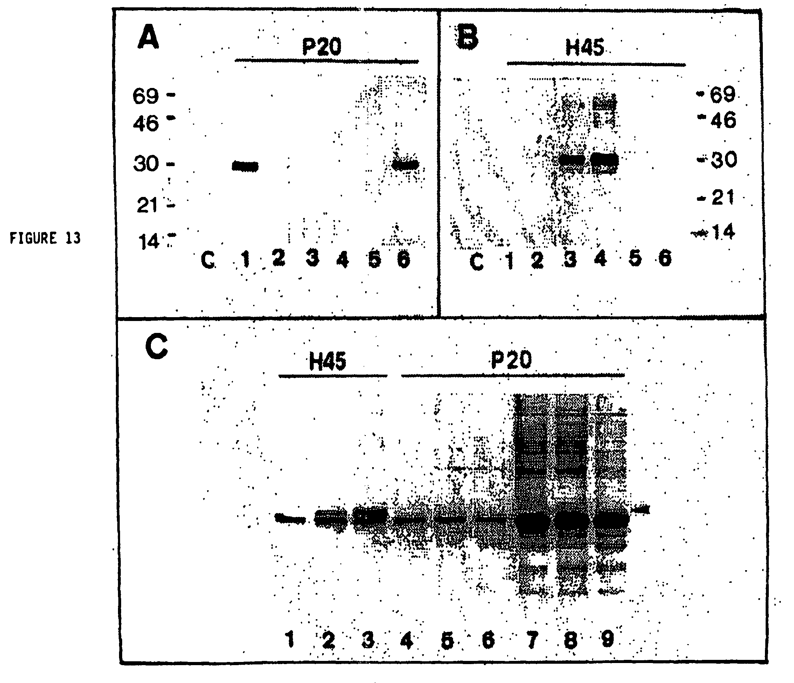

**Figure 13** - Endospermes des plants de maïs transgéniques accumulant de la γ-zéine enrichie en lysine
   A et B: SDS-page et Immunoblot utilisant l'antisérum αPL
      A) 10 µg de protéine par piste (transformants avec le construit H45γZ)
         piste C: extrait protéique des endospermes d'hybrides B73×A188 (contrôle)
         piste 1: A1
         piste 2: B1
         piste 3: B2
         piste 4: C1
         piste 5: D1
         piste 6: D2
      B) 1 µg de protéine par piste (transformants avec le construit P20γZ)
         piste C: contrôle
         piste 1: A1
         piste 2: A2
         piste 3: B1
         piste 4: C1
         piste 5: D1
         piste 6: E1
      C) SDS-page et coloration à l'argent (3 transformants P20γZ et 3 transformants H45γZ)

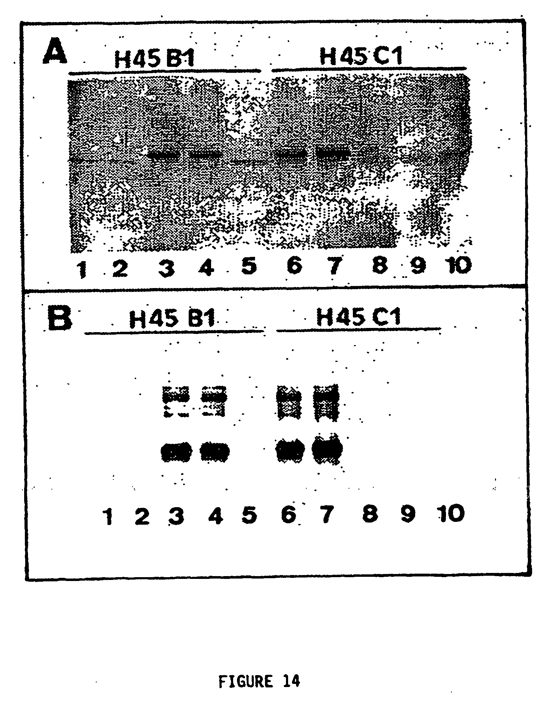

**Figure 14 -** Contenu en γ-zéine enrichie en lysine par grain (transformants 45γZ B1 et C1)
   A: coloration à l'argent ; 10 µg de protéine par piste
   B: immunoblot utilisant l'antisérum αPL ; 1 µg de protéine par piste
      Pistes 1 à 5: extraits protéiques de différents endospermes du transformant 45γZ B1.
      Pistes 6 à 10: extraits protéiques de différents endospermes du transformant 45γZ C1.
**Figure 15:**
   A) Contenu en γ-zéine enrichie en lysine de 10 grains (transformant 45γZ C1) utilisant l'antiserum αPL 1 µg de protéine par piste
      Pistes 1 à 10: extrait d'endospermes de 10 descendants
   B) Immunoblot des extraits protéiques des endospermes 1 à 5 présents en A) et marqués avec l'antisérum αG2; 2 µg de protéine par piste.

### EXEMPLES

### A) Préparation de γ-zéines modifiées enrichies en lysine et expression de ces protéines modifiées suivie de l'accumulation dans les corps protéiques des cellules de l'endosperme de maïs

La γ-zéine est une protéine de réserve du maïs, ayant un poids moléculaire de 28 kD, riche en soufre, qui est accumulée dans les cellules de l'endosperme avec les α-et β-zéines, dans les corps protéiques dérivés du réticulum endoplasmique granulaire (ER) (Ludevid et al., 1984. Plant Mol. Biol. 3, 227-234 ; Lending et al., 1984. Plant Cell 1, 1011-1023). La séquence d'acides aminés déduite de la séquence nucléotidique de l'ADNc (Prat et al., 1985, Nucl. Acids Res. 13, 1493-1504) et des clones génomiques (Boronat et al., 1986, Plant Sci. 47, 95-102) montre que la γ-zéine n'a pas d'homologie avec les polypeptides de type α-zéine. Bien que la γ-zéine soit codée par 1 à 2 gènes par génome haploïde (Boronat et al., 1986, Plant Sci. 47, 95-102) elle représente 10-15% de la totalité des protéines des endospermes de maïs. L'expression du gène de la γ-zéine dans des systèmes hétérologues tels que les oocytes de Xenopus (Torrent et al., 1994, Planta 192, 512-518) et dans Arabidopsis thaliana (Geli et al., 1994, Plant Cell 6, 1911-1922), indique que les polypeptides de γ-zéine restent stables et sont capables en tant que tels de former des corps protéiques dérivés du réticulum endoplasmique, à l'intérieur des cellules. De plus, des analyses par délétion de différents domaines structuraux de la γ-zéine ont montré que la séquence N-terminale incluant le domaine répété riche en proline, était responsable de la rétention de la γ-zéine dans le réticulum endoplasmique et que le domaine C-terminal riche en cystéine était responsable de la formation des corps protéiques. Le domaine Pro-X ne semblait pas en relation avec la stabilité de la protéine ni avec sa localisation ciblée (Geli et al., 1994, Plant Cell 6, 1911-1922).

### Matériel et Méthodes

### Matériel végétal

Après une stérilisation en surface (1) des graines de stade 17 DAP ("days after pollination") du maïs de sélection W64A ont été disséquées à la main et la couche de péricarpe et d'aleurone a été séparée des endospermes. Des sections tangentielles ont été réalisées de façon à exposer une grande partie de la surface sub-aleuronique. Si nécessaire, des embryons ont été isolés et des feuilles de plantes âgées de 7 jours ont été disséquées pour retirer le tissu épidermique. Après dissection, les échantillons ont été déposés dans des boites de Petri sur des papiers filtres humidifiés avec un milieu MS (Murashige et Skoog, 1962, Physiol. Plant. 15, 473-497).

### Constructions plasmidiques

Un premier ensemble de plasmides, pKSG2, pHbP2, pPbP4 et pNaN1 a été obtenu pour permettre l'introduction de sites de restriction dans le gène codant pour la γ-zéine. pKSG2 et pHbP2 ont été construits selon la description décrite dans la publication de Torrent M. et al. (Planta (1994) 192: 512-518). Le plasmide pKSG2 contient la séquence codante de la γ-zéine.

Le plasmide pHbP2 est obtenu à partir de pKSG2 et contient une séquence codant pour une γ-zéine mutée dans laquelle le domaine Pro-X de la protéine a été délété.

Le plasmide pPbP4 a été obtenu à la suite de deux étapes de clonage : (i) le fragment de restriction Sall-Pvull de 350 pb de pKSG2 a été cloné dans un plasmide Bluescript (pBSKS, Stratagene, La Jolla, California USA) restreint avec Sall et EcoRV (pKSC4) et (ii) le fragment de restriction Pvull-Xbal de 600 bp de pKSG2 a été cloné dans les sites de restriction Smal-Xbal de pKSC4. La nouvelle construction pPbP4 contient un site de restriction utile EcoRI juste avant le domaine P-X de la séquence codante de la γ-zéine.

Le plasmide pNaN1 a également été obtenu à l'aide de deux étapes de clonage: (i) le fragment Nael-Xbal de 250 bp de pKSG2 a été cloné dans le plasmide pBSKS restreint avec EcoRV-Xbal (pKSC8) et (ii) le fragment de restriction Nael-Hindlll de 700 bp (à extrémités franches) de pKSG2 a été cloné dans le site de restriction Hindlll de pKSC8. La nouvelle construction, pNaN1, contient des sites de restriction Clal et Hindlll à une position située à 15 nucléotides avant le codon stop de la γ-zéine.

Deux oligonucléotides synthétiques répondant aux séquences suivantes : SEQ ID N°1 :
5'CGATGAATTCAAACCAAAGCCAAAGCCGAAGCCAAAAGAATTCA3', et sa séquence inverse appelée SEQ ID N°2, dont la séquence est la suivante :
5'AGCTTGAATTCTTTTGGCTTCGGCTTTGGCTTTGGTTTGAATTCAT3' codant pour des séquences riches en lysine désignées K(P-K)₄, ont été hybridées, digérées avec EcoRI et clonées dans un site EcoRI de pHbP2 et pPbP4. Trois clones ont été sélectionnés : pPo2 et pHo3 contenant la séquence codante pour K(P-K)₄ et pHo4 comprenant la forme tronquée de la séquence codant pour le γ-zéine contenant un tandem 2K(P-K)₄ (sous forme d'une séquence K (P-K)₄ EF K(P-K)₄) de la séquence codante riche en lysine. Les mêmes oligonucléotides hybridés ont été digérés avec des enzymes ClaI-HindIII et clonés dans le plasmide pNaN1 restreint à l'aide des mêmes enzymes. Le clone sélectionné, pNo1, contenait la séquence codant pour la séquence riche en lysine K(P-K)₄ à l'extrémité N-terminale de la γ-zéine modifiée correspondante.

Pour la transformation transitoire de l'endosperme, les séquences codant pour la γ-zéine modifiée de pPo2 et pHo3 ont été insérées sous la forme de fragments HincII-NheI dans des sites SmaI-XbaI de pDH51 (Pietrzah et al., 1986, Nucl. Acid Res. 14, 5857-5868) contenant le promoteur 35S du virus de la Mosaïque du chou-fleur (CaMV). La construction pP20γZ obtenu par l'insertion décrite ci-dessus des fragments Hincli-Nhel dans les plasmide pDH51 contient la séquence codante de la γ-zéine enrichie en lysine (Figure 8) ainsi que les signaux de la séquence 35S du virus CaMV pour la formation de l'extrémité 3' et la polyadénilation. La séquence codante chimère P20γZ a été construite à partir de la région codante de la γ-zéine contenu dans le plasmide pKSG2 après différentes étapes de clonage. Le promoteur de 1,7 kb de la γ-zéine (Reina et al. 1990, Nucl. Acids Res. 18, 6426) a été inséré dans les extrémités franches d'un fragment HindII-PvuI dans pHo4 et pNo1 restreints avec Xho1 et obtenus avec des extrémités franches. Les constructions pH45γZ et pN13γZ ont respectivement été obtenues.

Les nouvelles constructions, respectivement appelées pP20γ-Z, pH30γZ, pH45γZ et pN13γZ ont été utilisées dans des expériences de bombardement biolistique.

Pour étudier la spécificité de différents promoteurs vis-à-vis des tissus végétaux, deux constructions. p1.7γZGUS et pCaMV35SLUC ont été utilisées. p1.7γZGUS a été obtenue par insertion du promoteur de la γ-zéine de 1,7 kb (Hindlll-Pvul) dans un plasmide dérivé de pPuC18 contenant le gène GUS et les signaux NOS de polyadénilation en 3' de pBI 101.1 (Jefferson et al., 1987, Embo. J. 6, 3901-3907). pCaMV35SLUC a été obtenu par insertion du gène codant pour la luciférase (LUC) de pAHC18 (Bruce et al., 1989, P. H. 86. 9692-9696) dans le polylinker de pDH51 (Pietrzak et al., 1986, Nucl. Acids Res. 18, 6426).

### Analyse in-vitro

Les plasmides dérivés de pBSKS contenant les séquences codantes de la γ-zéine (pKSG2) et de la γ-zéine riche en lysine (pPo2, pHo3, pHo4 et pNo1) ont été transcrits in-vitro suivant les protocoles standards (Sambrook et al., 1989, Molecular Cloning: A laboratory manual, 2nd. ed., Cold Spring Harbor Laboratory Ed., Cold Spring Harbor, New York). Une traduction in-vitro et une translocation des transcrits synthétiques ont été réalisées selon la technique de Torrent et al., (1994, Panta 192, 512-518), à l'exception des microsomes canins (CM) utilisés qui provenaient de Promega (Madison, Wis., USA). L'immunoprécipitation des produits traduits a été effectuée essentiellement selon la méthod de Borgese et Gaetani (1980) en utilisant un sérum de lapin anti-γ-zéineα-G2, (Ludevid et al., 1985, Plant Sci. 41, 41-48) et un antisérum αPL. αPL est un antisérum polyclonal de lapin obtenu contre le peptide synthétique EFK(P-K)₈EF. Ce peptide a été synthétisé en utilisant la technique de synthèse en phase solide décrite par Celma et al., 1992.

### Bombardement de microprojectile

L'ADN plasmidique a été absorbé sur des particules d'or (1,0 µm, Bio-Rad, Lab., Richmond, CA, USA) selon un protocole décrit par Kikkert (Plant Cell, 33:221-226, 1993). Toutes les cibles ont été bombardées deux fois, en utilisant le dispositif BioRad Biolistic PDS-1000/He. Les cibles ont été positionnées 8 cm derrière un écran stoppant les macroporteurs, qui étaient positionnés 1 cm au dessous du disque de rupture 900 PSI. Après le bombardement, les échantillons ont été incubés pendant 24 heures à 26°C dans le noir. Des témoins étaient constitués par les cibles bombardées avec des microprojectiles dépourvus d'ADN.

### Tests enzymatiques

Les tissus bombardés avec les plasmides p1.7γZGUS et pCaMV35SLUC ont été homogénéisés sur de la glace dans un tampon contenant 25mM Tris, à pH 7,8, 2mM DTT, 10% de glycérol et 1% de Triton X-100. Après centrifugation à 12.000xg pendant 5 minutes, les surnageants ont été décantés et la protéine soluble totale dans les extraits a été quantifiée en utilisant le test Bradford (Bio-Rad). L'activité GUS a été testée par analyse fluorimétrique selon la description de Jefferson (1987) en utilisant la 4-méthyl-ombélliféryl-β-D-glucuronide (MUG) en tant que substrat. L'activité LUC a été déterminée en utilisant le système de test Luciférase (Luciferase Assay System Kit) diffusé par Promega, selon les instructions du fabriquant.

### Extraction des protéines de réserve et analyse sur gel des protéines

Des endospermes transformés avec pP20γZ, pH20γZ,- pH45γZ et pN13γZ ont été réduits a l'état de farine et les α-zéines ont été extraites à l'aide de trois séries de solvants contenant 70% d'éthanol. La farine résiduelle a été séchée à l'air, et les protéines totales ont été extraites avec un tampon contenant 0,25 M Tris-HCl, pH 6,8, 4% sodium dodecyl sulfate (SDS) et 5% 2-mercaptoethanol, pendant 1 heure à température ambiante. Les extraits de protéines ont été analysés par SDS-PAGE et immunoblot selon la description de Ludevid et al., 1985. Des feuilles de nitrocellulose ont été intubées avec de l'antisérum αPL (dilution 1:500) et de la peroxidase de Raifort conjuguée avec un anticorps secondaire (ECL Western Blotting System, Amersham, Buckinghamshire, UK) a été utilisée pour la détection de la protéine.

### Analyse de l'expression de l'ARN

L'ARN total a été extrait selon la description de Logemas et al., 1987. L'ADN complémentaire (ADNc) a été préparé en utilisant la transcriptase réverse et l'oligo dT de Gibco BRL (Gaithersburg, MD, USA) selon les instructions du fabriquant et cet ARN a été amplifié par une réaction de PCR. Des oligonucléotides amorces utilisées pour la PCR étaient des séquences de 20-mers correspondant aux extrémités de 5' et 3' du gène de structure de la γ-zéine. Des protocoles standards ont été utilisés pour la préparation de sondes marquées au ³²P, et pour l'analyse sur gel de l'ADN (Sambrook et al. Molecular Cloning: A laboratory manual, 2nd. ed., Cold Spring Harbor Laboratory Ed., Cold Spring Harbor, New York) en utilisant un oligonucléotide synthétique codant pour une séquence riche en lysine (voir ci-dessus) en tant que sonde.

### Isolement des corps protéiques et traitement par la protéase

Ces protocoles ont été décrits antérieurement (Torrent et al., Planta, 180: 90-95, 1989).

### Microscopie électronique

Les corps protéiques des endospermes de type sauvage et des endospermes transformés par pP20γZ ont été fixés avec 2,5% de paraformaldéhyde dans 20 mM de tampon phosphate à pH7,2, pendant 1 heure à température ambiante, et transformés selon la description de Geli et al., 1994, Plant Cell 6. 1911-1922) toutefois en utilisant un antisérum α-PL et un colloïde d'or et de protéine A ayant un diamètre de 15 nm. Pour le marquage double, des sections ultra fines ont été d'abord incubées avec α-PL et le colloide d'or et de protéine A (15 nm de diamètre) a été utilisé pour la détection de l'anticorps. Après lavage, des sections ont été incubées avec 0,15 mg/mL de protéines A pendant 20 minutes pour saturer les immunoglobulines et finalement les grilles ont été incubées avec des séra α-G2 ou α-Z1 et le colloïde or/protéine A (diamètre 5 nm) a été utilisé pour la détection de l'anticorps. α-Z1 est un antisérum polyclonal de lapin dirigé contre l'α-zéine obtenu selon la description de Ludevid et al., 1985. Plant Sci. 41, 41-48).

### Résultats

### Construction des γ-zéines riches en lysine

Les inventeurs avaient démontré l'importance du domaine répété riche en proline ("proline-rich repeat") et du domaine C-terminal riche en cystéine pour la rétention dans le réticulum endoplasmique de la γ-zéine et la formation des corps protéiques contenant ces protéines dans les cellules de feuilles de Arabidopsis (Geli et al., 1994, Plant Cell 6, 1911-1922). Sur la base de ces résultats préalables, la possibilité d'insérer des séquences riches en lysine dans différents domaines de la γ-zéine, de façon à créer une γ-zéine modifiée correctement ciblée et accumulée dans les cellules de l'endosperme a été investiguée afin d'améliorer les qualités nutritives du maïs.

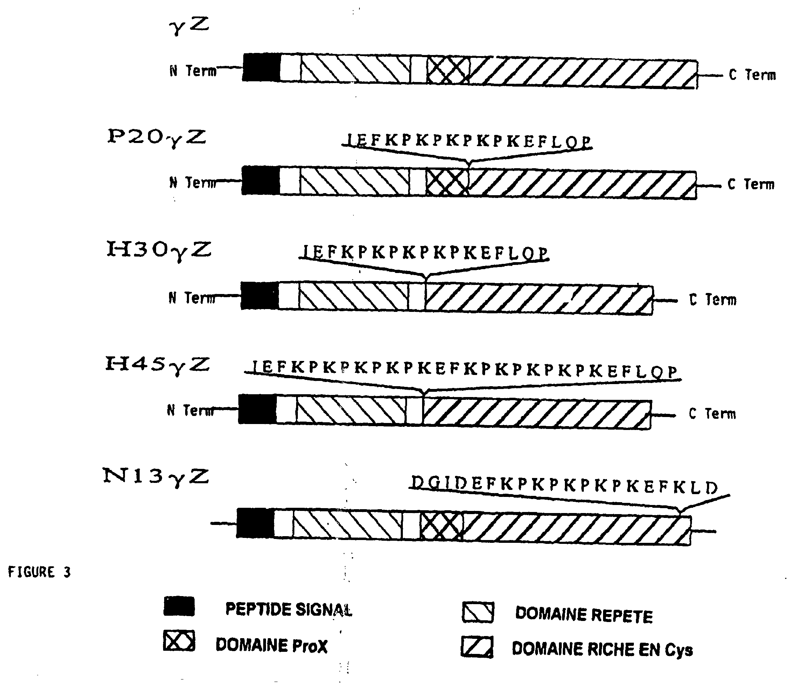

Les inventeurs ont à présent construit des gènes modifiés de la γ-zéine par introduction d'oligonucléotides synthétiques codant pour des séquences riches en lysine dans différents sites de la séquence codante de la γ-zéine. Des constructions de γ-zéine modifiée ont été créées de façon à éviter le placement des séquences codantes riches en lysine dans les domaines constitués par le domaine répété en tandem et le domaine riche en cystéine. Des modifications de la séquence codante de la γ-zéine ont été effectuées dans la séquence correspondant au domaine Pro-X. De plus pour minimiser une altération du repliement de la protéine, les séquences riches en lysine (P-K)ₙ ont été définies pour imiter la séquence du domaine Pro-X. Comme on le voit sur la figure 3, dans la protéine P20γZ, une séquence K(P-K)₄ a été introduite après la région Pro-X et dans la protéine H30γZ et dans la protéine H45γZ, des séquences d'acides aminés comprenant K(P-K)₄ et 2K(P-K)₄ respectivement remplacent le domaine Pro-X de la γ-zéine (γZ, Fig. 3). Pour explorer si l'extrémité C-terminale était un site permissif pour l'introduction des séquences riches en lysine, une protéine supplémentaire N13γZ, a été créée par l'insertion d'une séquence contenant K(P-K)₄ cinq acides aminés en amont de l'extrémité C-terminale (Fig.3).

### Activité du promoteur de la γ-zéine dans l'endosperme de maïs transformé

Pour déterminer si des γ-zéines riches en lysine pouvaient être exprimées dans les cellules de l'endosperme, on a tout d'abord recherché un promoteur efficace et un système de transformation. Un promoteur de la γ-zéine, contenant une séquence en amont de 1,7 Kb (Reina et al., 1990, Nucleic Acid Research, vol. 18, p. 6426) et le promoteur CaMV contenant 625 bp de la séquence en amont de la protéine 35S du virus de la mosaïque du chou-fleur CaMV ont été testés. Jusqu'à présent aucune information n'était disponible sur l'analyse fonctionnelle des fusions de gènes avec le promoteur de la γ-zéine dans les plantes monocotylédones transgéniques. Pour analyser l'activité et la spécificité tissulaire du promoteur de la γ-zéine, deux gènes chimères ont été construits (voir Fig. 5). l'expression transitoire par bombardement biolistique (Klein et al., 1988 PNAS 85:4305) a été utilisée comme procédure de transformation du maïs pour l'analyse du promoteur aussi bien que pour des expériences d'expression des γ-zéines riches en lysine. Des endospermes de maïs de stade 17 DAP (17 jours après la polynisation) (le péricarpe et les cellules de la couche aleuroniques étaient enlevés), des embryons (17 DAP) et des jeunes feuilles (âgées de 10 jours) ont été bombardés avec des projectiles d'or recouverts avec de l'ADN plasmidique contenant les deux constructions. La figure 5 montre les activités de β-glucuronidase (GUS) et de luciférase (LUC) présentes dans les trois tissus testés : l'endosperme, l'embryon et la feuille, par rapport à l'expérience témoin. On doit noter que les résultats correspondent à la moyenne d'au moins 3 expériences indépendantes réalisées. Toute l'activité GUS sous le contrôle du promoteur de la γ-zéine était restreinte à l'endosperme, puisque aucune expression GUS n'a été détectée dans l'embryon et dans les feuilles. De plus, les endospermes bombardés ont été colorés histochimiquement pour déterminer le nombre de clusters cellulaires exprimant la protéine GUS. Le profil de coloration corroborait les résultats précédents, GUS était fortement exprimé dans les endospermes (le nombre moyen de clusters cellulaires colorés pour GUS par endospermes était de 150) et des tâches bleues n'ont pas été détectées dans l'embryon et dans les feuilles. Au contraire, le promoteur CaMV 35S conférait une activité LUC dans tous les tissus testés (Fig. 5), mais il existait cependant des différences quantitatives entre l'activité relative de l'enzyme dans les feuilles et les embryons par rapport à l'endosperme. Ces différences pouvaient être attribuées à une variabilité intrinsèque de l'activité constitutive du promoteur CaMV parmi les différents tissus du mais ou à une pénétration faible des particules recouvertes par l'ADN dans les cellules mésophyles contenant un système vacuolaire important. Il existe dans l'état de la technique des preuves selon lesquelles le promoteur CaMV aurait habituellement une faible activité dans des cellules de plantes monocotylédones. (Fromm et al., 1985, Proc. Natl. Acad. Sci. USA 82, 5824-5828) ; cependant les inventeurs ont montré ici une forte activité du promoteur CaMV dans des cellules d'endospermes. Ceci a permis de conclure que l'activité du promoteur de la γ-zéine et du promoteur CaMV 35S était très forte dans les endospermes de maïs et ainsi que les deux promoteurs pouvaient s'avérer utiles pour contrôler l'expression de la protéine dans ce tissu.

Pour déterminer si les protéines mutantes codées par les constructions étaient compétentes avec la fonction de translocation membranaire, des expériences de transcription-traduction in-vitro ont été effectuées en présence de microsomes pancréatiques de chien. Les transcrits synthétiques de chaque construction ont été traduits et la translocation à travers les membranes de microsomes testée par recherche de la protection vis-à-vis de la digestion par la protéinase K Ces résultats rapportés à la figure 4A indiquent que les poids moléculaires apparents des polypeptides synthétisés in-vitro reflètent les mutations introduites (Fig. 4A, lignes 1, 5, 9 et 13). En présence des microsomes et de la protéinase K, des peptides de faible poids moléculaire n'ont pas été observés, indiquant que les chaînes polypeptidiques complètes des γ-zéines modifiées étaient transportées à travers les membranes microsomales (Fig. 4A, lignes 3, 7, 11 et 15). En comparant le résultat de la translocation des quatre γ-zéine modifiées, on a observé que la protéine H45γZ (Fig. 4A, ligne 11), qui contenait l'insertion de 10 acides aminés de type lysine, a subi une translocation inférieure aux autres protéines. Il semble que les résidus chargés négativement pourraient interférer dans une certaine proportion, sur l'efficacité de la translocation. L'anticorps polyclonal αG2, dirigé contre la γ-zéine (Ludevid et al., 1985, Plant Sci. 41, 41-48) ne pouvant être utilisé pour distinguer entre la γ-zéine de type sauvage et les γ-zéines modifiées, un anticorps αPL dirigé contre un peptide synthétique contenant la séquence d'acides aminés riche en lysine a été préparé. On a ensuite testé si des protéines modifiées synthétisées in-vitro étaient reconnues à la fois par les anticorps αG2 et les anticorps αPL. Cette expérience est illustrée à la figure 4B et la figure 4C où les transcrits synthétiques de la γ-zéine, P20γZ, H30γZ, H45γZ et N13γZ étaient traduits in-vitro et dans lesquels les produits de traduction étaient immunoprécipités avec αPL (Fig. 48) et avec αG2 (Fig. 4C). Ces résultats indiquent que les γ-zéines riches en lysine étaient reconnues par les deux anticorps (voir Fig. 4B et C, lignes 2 à 5) et que la γ-zéine était seulement reconnue par le sérum αG2 (Fig. 48 et C, ligne 1). Ainsi la spécificité des anticorps αPL pour les protéines modifiées a permis de distinguer la γ-zéine riche en lysine de la γ-zéine endogène lorsque les gènes modifiés étaient exprimés dans des cellules d'endosperme. Prises ensemble ces expériences ont montré que la présence de séquences riches en lysine ne perturbait pas la fonction de translocation à travers la membrane ou le comportement immunologique de la γ-zéine.

### Analyse de l'expression des γ-zéines enrichies en lysine dans les endospermes de maïs

Pour explorer si la γ-zéine modifiée riche en lysine était exprimée et accumulée dans les cellules de l'endosperme, des graines de stade 17 DAP ont été bombardées avec l'ADN contenant les séquences codant pour la protéine des quatre constructions (Fig. 3) sous le contrôle du promoteur CaMV (P20γZ et H20γZ) et du promoteur de la γ-zéine (H45γZ et N13γZ). Des constructions comprenant des promoteurs anti-sens ou dépourvues de promoteurs ont été utilisées comme témoins. Après 24 heures de transformation des endospermes, les protéines totales ont été extraites et l'expression des γ-zéines modifiées a été testée par immunoblotting en utilisant l'anticorps αPL. La figure 6A montre que les gènes chimères de γ-zéine contenant l'insertion (Pro-Lys)ₙ après le domaine Pro-X (P20γZ) ou le remplaçant (H45γZ et H30γZ) étaient fortement exprimés et les produits de traduction accumulés efficacement dans les cellules de l'endosperme (Fig. 6A, lignes 3, 4 et 5). Pour chaque ligne, les extraits de protéine correspondaient à 1/3 environ d'un endosperme bombardé, ce qui a permis d'estimer que la quantité de protéines modifiées P20γZ, H30γZ et H45γZ par endosperme atteignait le niveau du nanogramme. De plus, aucune différence quantitative entre les niveaux d'expression des gènes chimères sous le contrôle du promoteur CaMV et du promoteur de la γ-zéine n'a été observée, confirmant les résultats ci-dessus décrits obtenus avec les protéines marqueurs GUS et LUC (Fig. 5). On a noté que l'anticorps αPL reconnaissait une protéine d'environ 30 kD présente dans les extraits de protéine totale, même dans les endospermes non transformés (voir la bande faible présente dans les quatre lignes de la figure 6A). Une procédure d'extraction séquentielle de protéine a permis de constater que cette protéine n'était pas une protéine de réserve soluble dans un milieu aqueux.

Comme le montre la figure 6A (ligne 2) aucune trace de la protéine N13γZ n'a été détectée, indiquant que le gène chimère correspondant n'était pas exprimé dans les cellules de l'endosperme ou que la protéine N13γZ était dégradée. Des ARNs des endospermes transformés avec les ADNs codant pour les protéines H45γZ et N13γZ ainsi que les ARNs des endospermes non transformés ont été analysés. A partir des ARNs totaux, les ADNc ont été préparés et amplifiés par PCR en utilisant des amorces spécifiques. La Fig. 6B montre l'analyse en Southem blot de trois échantillons d'ADNc hybridés avec un oligonucléotide codant pour une séquence K(Pro-Lys)₄ utilisée comme sonde. Les résultats indiquent que le gène N13γZ était correctement exprimé (Fig. 6B, ligne 3). La présence de bandes dans les échantillons H45γZ et N13γZ, mais pas dans les endospermes non transformés, a suggéré que la protéine N13γZ était dégradée pendant les 24 heures de l'incubation. A partir de ces observations, les inventeurs ont conclu que le site d'insertion des séquences riches en lysine était critique pour la stabilité de la γ-zéine modifiée.

### La γ-zéine enrichie en lysine est accumulée dans le corps protéiques

En dehors de la teneur en lysine des séquences Pro-X, les protéines P20γZ, H30γZ et H45γZ avaient des caractéristiques communes avec la γ-zéine de type sauvage: le peptide signal, le domaine répété en tandem N-terminal et la région riche en cystéine C-terminale étaient apparentées. Il a semblé important de déterminer si ces domaines restaient totalement fonctionnels préservant le ciblage et la formation des corps protéiques ou si les séquences riches en lysine créaient un environnement spécial dans lequel ces propriétés pouvaient être perturbées. Pour tester ceci, on a investigué si les γ-zéines modifiées étaient capables de s'accumuler dans les corps protéiques. Un fractionnement sub-cellulaire a été réalisé avec les endospermes transformés. Les homogénats d'endospermes bombardés ont été chargés sur des gradients de sucrose discontinus (20 %. 50 % et 70 % de sucrose) et toutes les fractions collectées ont été analysées par immunoblotting. P20γZ, H30γZ et H45γZ sédimentés sur la fraction des corps protéiques et aucune quantité significative de ces protéines n'a été détectée, soit dans le surnageant, soit dans la fraction microsomale (Fig. 7A, lignes 2, 2 et 3). Bien que dans les expériences faites préalablement in-vitro (Fig. 4A) aient permis de constater que les γ-zéines modifiées nouvellement synthétisées subissaient une translocation dans les microsomes canins, on a testé ici si les protéines modifiées exprimées in-vivo dans des cellules d'endosperme, subissaient une translocation dans la membrane du réticulum endoplasmique et restaient à l'intérieur des corps protéiques dérivés du réticulum endoplasmique. Pour cette raison, des corps protéiques isolés ont été soumis à une digestion par la protéinase K dans des tampons isotoniques (contenant 20 % de sucrose) ou après un choc osmotique dans l'eau (Fig. 78). Les protéines protégées contre la dégradation protéolytique par des enzymes peuvent être entourées par une membrane et un traitement par des solutions détergeantes ou hypotoniques a résulté dans la digestion des protéines (Walter et Blobel, 1983, Method Enzymol. 96, 84-93). Une comparaison des intensités de bandes, après digestion par la protéinase K dans des milieux comprenant du sucrose ou de l'eau, a permis de révéler que les protéines P20γZ, H30γZ et H45γZ étaient protégées de la digestion dans des tampons isotoniques (lignes 1, 3 et 5) mais étaient partiellement digérées dans l'eau (lignes 2, 4 et 6).

L'expression des gènes modifiés de la γ-zéine dans les cellules de la couche du sub-aleurone de l'endosperme par bombardement biolistique a permis d'observer que des γ-zéines riches en lysines étaient largement accumulées à l'exception du cas où les séquences riches en lysine étaient positionnées 5 résidus en amont de l'extrémité C-terminale du polypeptide de la γ-zéine. A partir de cette expression et d'études immunocytochimiques sur des corps protéiques isolés, les inventeurs ont démontré que des γ-zéines riches en lysine sont correctement accumulées dans ces organites et sont co-localisées avec les protéines endogènes γ-zéine et α-zéine.

Des corps protéiques isolés à partir des endospermes P20γZ ont été examinés par marquage de type immunogold et microscopie électronique. Sur des sections ultra fines incubées avec l'anticorps αP-L (Fig. 8A), le marquage à l'or a été détecté à l'intérieur des corps protéiques, indiquant que la protéine P20γZ riche en lysine était accumulée à l'intérieur de ces organites. Dans des sections incubées seulement avec l'anticorps αP-L, l'immunomarquage avait lieu uniquement sur quelques corps protéiques (contenant la γ-zéine riche en lysine), la plus grande partie des corps protéiques isolés n'était pas immunomarquée avec des anticorps αP-L parce qu'ils correspondaient à des cellules d'endosperme non transformées. Pour déterminer si la γ-zéine riche en lysine était co-localisée avec les polypeptides α-zéines et γ-zéines, un double marquage en immuno-électromicroscopie a été réalisé sur des corps protéiques isolés en utilisant les anticorps αZ et αP-L (Fig. 8B) et αG2 et αP-L (Fig. 8C et D). La figure 8B montre une micrographie de la section transversale de deux corps protéiques marqués avec l'anticorps αP-L (particule d'or de 15 nm) et avec l'anticorps αZ (particule d'or de 5 nm). Le résultat de l'immunocoloration a montré que la protéine P20γZ est accumulée dans les corps protéiques et co-localisée avec l'α-zéine (voir l'étendue du marquage de l'α-zéine sur toute la surface du corps protéique). Par ailleurs, des sections tangentielles (Fig. 8B, voir les flèches) et transversales (Fig. 8D) des corps protéiques ont été incubées avec l'anticorps αP-L (particule d'or 15 nm) et avec l'anticorps αG2 (particule d'or de 5 nm). Dans les deux cas, la protéine P20γZ était co-localisée avec les polypeptides γ-zéines. On a pu noter que les sections tangentielles du corps protéique (Fig. 8A, C, voir les flèches) étaient facilement distinguées des sections transversales du corps protéique dans la mesure où les premiers présentaient une densité plus forte en électrons et que le marquage de la γ-zéine était étendu sur toute la section. Au contraire les sections transversales présentaient une densité inférieure et le marquage de la γ-zéine était localisé sur la membrane entourant le corps protéique. Dans les deux cas, la localisation du marquage de la γ-zéine riche en lysine suivait celle de la γ-zéine endogène.

### B) Préparation de plantes génétiquement modifiées, exprimant des γ-zéines riches en lysine

### 1) Obtention et utilisation de cal de maïs comme cible pour la transformation génétique.

La transformation génétique du maïs, quelle que soit la méthode employée (electroporation; Agrobacterium, microfibres, canon à particules) requiert généralement l'utilisation de cellules indifférenciées en divisions rapides ayant conservé une aptitude à la régénération de plantes entières. Ce type de cellules compose le cal friable embryogène (dit de type II) de maïs.

Ces cals sont obtenus à partir d'embryons immatures de génotype Hi II ou (A188 x B73) selon la méthode et sur les milieux décrits par Armstrong (Maize Handbook ; (1994) M. Freeling, V. Walbot Eds ; pp 665-671). Les cals ainsi obtenus sont multipliés et maintenus par repiquages successifs tous les quinze jours sur le milieu d'initiation.

Des plantules sont ensuite régénérées à partir de ces cals en modifiant l'équilibre hormonal et osmotique des cellules selon la méthode décrite par Vain et al. (Plant Cell Tissue and Organ Culture (1989 18 : 143-151). Ces plantes sont ensuite acclimatées en serre où elles peuvent être croisées ou autofécondées.

### 2) Utilisation du canon à particules pour la transformation génétique du maïs.

Le paragraphe précédent décrit l'obtention et la régénération des lignées cellulaires nécessaires à la transformation ; on décrit ici une méthode de transformation génétique conduisant à l'intégration stable des gènes modifiés dans le génome de la plante. Cette méthode repose sur l'utilisation d'un canon à particules identique à celui décrit par J. Finer (Plant Cell Report (1992) 11 :323-328) ; les cellules cibles sont des fragments des cals décrits dans le paragraphe 1. Ces fragments d'une surface de 10 à 20 mm² ont été disposés, 4h avant bombardement, à raison de 16 fragments par boite au centre d'une boite de petri contenant un milieu de culture identique au milieu d'initiation, additionné de 0,2 M de mannitol + 0,2M de sorbitol. Les plasmides portant les gènes à introduire sont purifiés sur colonne Qiagen® en suivant les instructions du fabricant Ils sont ensuite précipités sur des particules de tungsten (M10) en suivant le protocole décrit par Klein (Nature (1987) 327 :70-73). Les particules ainsi enrobées sont projetées vers les cellules cibles à l'aide du canon et selon le protocole décrits par J. Finer (Plant Cell Report (1992) 11 : 323-328).

Les boites de cals ainsi bombardées sont ensuite scellées à l'aide de Scellofrais® puis cultivées à l'obscurité à 27°C. Le premier repiquage a lieu 24h après, puis tous les quinze jours pendant 3 mois sur milieu identique au milieu d'initiation additionné d'un agent sélectif dont la nature et la concentration peuvent varier selon le gène utilisé (voir paragraphe 3). Les agents sélectifs utilisables consistent généralement en composés actifs de certains herbicides (Basta®, Round up®) ou certains antibiotiques (Hygromycine, Kanamycine...).

On obtient après 3 mois ou parfois plus tôt, des cals dont la croissance n'est pas inhibée par l'agent sélectif, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boite bombardée.

Ces cals sont identifiés, individualisés, amplifiés puis cultivés de façon à régénérer des plantules (cf. paragraphe 1). Afin d'éviter toute interférence avec des cellules non transformées toutes ces opérations sont menées sur des milieux de culture contenant ragent sélectif.

Les plantes ainsi régénérées sont acclimatées puis cultivées en serre où elles peuvent être croisées ou autofécondées.

### 3) Utilisation du gène Bar pour la production plantes de maïs génétiquement modifiées ayant incorporé et exprimant le gène H45γZ

Le gène Bar de Streptomyces hygroscopicus code pour une phosphinotricine acyl transférase (PAT) qui inactive par acetylation la phosphinotricine-molecule active de l'herbicide Basta®. Les cellules portant ce gène sont donc rendues résistantes à cet herbicide et peuvent être sélectionnées par son intermédiaire. Pour la transformation des céréales la séquence codante du gène Bar est sous le contrôle d'une région régulatrice permettant une expression forte et constitutive dans les cellules végétales. Une telle région est avantageusement constituée par le promoteur et le premier intron du gène Actine de riz tels que décrits par Mc Elroy (Mol. Gen. Genet. (1991) 231 : 150-160).

Ce gène chimérique est cloné sur un plasmide permettant son amplification par Escherichia coli. Ce plasmide (pDM 302 Cao (Plant Cell Report (1992) 11:586-591) après amplification puis purification sur colonne Qiagen® peut être utilisé en transformation génétique du maïs en utilisant par exemple la méthode décrite dans l'exemple précédent. Dans ce cas les milieux de culture destinés à la sélection des cellules transformées sont additionnés de 2 mg/L de Phosphinotricine.

Pour l'introduction du gène H45γZ une technique dite de cotransformation est avantageusement utilisée : les gènes de sélection (Bar) et d'intérêt (H45γZ) sont portés par des plasmides indépendants. Dans le cas de l'utilisation du canon à particules on procède à une coprécipitation des plasmides sur les particules de tungstène, la quantité totale d'ADN précipité sur les particules restant identique à ce qu'elle est dans le protocole standard (5µg d'ADN pour 2,5 mg de particules) chaque plasmide représentant environ la moitié du poids total d'ADN utilisé.

L'expérience montre qu'avec cette méthode, la cointégration des plasmides dans les cellules végétales est l'évènement le plus fréquent (de l'ordre de 90%) c'est-à-dire que pratiquement chaque plante ayant intégré le gène Bar et sélectionnée par son intermédiaire portera aussi le gène H45γZ. Le niveau de coexpression (pourcentage de plantes sélectionnées exprimant le gène H45γZ) est habituellement de l'ordre de 70%.

Les gènes ainsi introduits sont généralement liés au sens génétique, le gène H45γZ peut ainsi avantageusement être suivi dans les descendances grâce à la résistance à l'herbicide qui lui est étroitement associée.

La quantité de protéine modifiée est déterminée par les méthodes décrites dans l'exemple A, notamment par immunoblotting sur extraits protéiques de grains de maïs immatures ou matures, prélevés en pool sur des plantes resistantes au BASTA.

### 4) Exemple explicitant l'étape d'introduction des transgènes et notamment du gène codant pour la H45γZ, pour la modification du phénotype opaque 2 du maïs

Amélioration de maïs opaque 2 par l'introgression du gène de γ-zéine enrichie en lysine.

Les plantes transformées décrites dans l'exemple précédent, présentent à la fois, une résistance au Basta et l'expression d'une γ-zéine enrichie en lysine sont utilisées. Leur pollen est utilisé pour féconder les plantes de maïs opaque 2 de la lignée W64Ao2 qui ne contient qu'un seul gène γ-zéine. Cette lignée a été obtenue auprès du Maize Stock Center. Les plantes de ces descendances F1 sont sélectionnées pour leur résistance au Basta et ensuite autofécondées. Les graines F2 ainsi produites sont analysées pour le phénotype opaque sur une table lumineuse et les grains opaque ou les grains vitreux sont semés et évalués pour la résistance au Basta. Dans le cas où tous les grains opaques sont sensibles au Basta, la démonstration est faite que l'introduction du gène γ-zéine enrichi en lysine dans la plante considérée, complémente le phénotype opaque 2.

Dans un deuxième temps, dans ces plantes Basta résistantes, des individus de génotype o2/o2 et ne possédant qu'un seul gène γ-zéine sur le chromosome 7 sont sélectionnés à l'aide des sondes moléculaires codant pour le gène Opaque 2 et pour la γ-zéine. Ces dernières révélant des fragments de restriction polymorphes et seuls les individus présentant le patron type de la lignée W64o2 sont retenus (Lopes M.A. et al, 1995, Mol. Gen. Genet. 19 : 247, 603-613).

Ces individus ont un contenu en lysine qui est en moyenne équivalent à celui du maïs o2, voire supérieur. A partir de ces individus, toute introgression dans des variétés ELITE, du caractère « forte teneur en lysine » est observée via la détermination de la résistance au BASTA et de la présence de l'allèle o2 détectée par RFLP.

### 5) Expression de γ-zéines enrichies en lysine dans Arabidopsis thaliana.

Pour obtenir une transformation stable, les constructions plasmidiques P20γZ et pH30γZ clonées dans le plasmide Bluescript KS (-) ont été insérées sous forme de fragments HincII/XbaI dans le vecteur binaire pBin19 (Bevan, M. Nucl. Acids Res. 12: 8711-8721 (1984)), contenant le promoteur 35S du virus de la mosaïque du chou-fleur (CaMV) et les signaux de formation de l'extrémité 3' et de polyadénylation du gène de l'octopine synthétase (ocs). Les nouveaux plasmides ont été appelés p19P20γZ et p19H30γZ (figure 12).

Les vecteurs binaires contenant les séquences codant pour les protéines P20γZ et H30γZ (p19P20γZ et p19H30γZ) ont été transférés dans la souche LBA4404 d'Agrobacterium tumefaciens. Des plantes Arabidopsis ecotype RLD ont été transformées selon la méthode décrite par Valvekens, D., Van Montagu, M. et Van Lijsebettens, M. ((1988) Proc. Natl.Acad.Sci.USA 85: 5536-5540). Pour chaque construction, 10 plantes transgéniques ont été criblées par analyse en immunoblot en utilisant un antisérum obtenu contre la γ-zéine (αG2, Ludevid et al. 1985). Les plantes contenant les taux les plus élevés (correspondant à environ 0.1% de la quantité totale de protéines présentes dans les feuilles d'Arabidopsis) de produits transgéniques dans la génération F1, ont été choisies pour obtenir la génération F2. Ces plantes ont également été sélectionnées pour l'expression de la protéine recherchée.

Des plantes transgéniques entières, sélectionnées dans un milieu contenant de la kanamycine ont été homogénéisées dans de l'azote liquide. Les protéines transgéniques ont été extraites sélectivement avec une solution contenant de l'éthanou /de l'acide chlorhydrique HCl 0,125 N dans une proportion de 3:1 (v/v), avec 5% de mercaptoéthanol et d'inhibiteurs de protéase. Les protéines extraites avec cette solution ont été précipitées dans 5 volumes d'acétone et analysées par SDS-PAGE et immunoblotting. Les extraits protéiques des plantes non transgéniques ont été utilisés en tant que contrôles. Les protéines résultant de l'insertion des séquences K(P-K)₄ dans la γ-zéine ont été exprimées correctement dans les plantes Arabidopsis thaliana en utilisant le promoteur constitutif 35 S du CaMV. Sur les immunoblots, les anticorps αG2 et αPL reconnaissaient des bandes d'électrophorèse correspondant aux protéines P20γZ et H30γZ. Ces bandes migraient avec des masses moléculaires apparentes conformes à celles que l'on avait préalablement observées dans les expériences de traduction / translocation in vitro (30 kD et 26 kD respectivement). Comme on l'a observé dans les plantes transgéniques Arabidopsis exprimant la γ-zéine (Geli et al. Plant Cell 6: 1911-1922 (1994)), les protéines P20γZ et H30γZ migraient sous la forme de deux bandes d'électrophorèse à savoir les bandes correspondant à 36 et 30 kD pour P20γZ et les bandes correspondant à 32 et 26 kD pour H30γZ. Les bandes supérieures pourraient correspondre à des produits ayant subi des modifications post-traductionnelles. Une telle modification post-traductionnelle n'a pas été détectée dans les endospermes de mais transformés. Ce résultat suggère que la modification apparaîtrait lorsque ces protéines sont exprimées dans un système hétérologue tel que Arabidopsis thaliana.

### 6) Expression de γ-zéines enrichies en lysine recombinantes dans du maïs.

### Méthode:

Après obtention des plantes transgéniques, on les croise avec une lignée mâle non transformée. Par conséquent, 50% des grains récoltés dans ce cas d'insertion unilocus seront transgéniques. Dans le but d'analyser les γ-zéines enrichies en lysine dans les plantes transgéniques, les protéines sont extraites à partir de 6 grains par transformant.

Les endospermes sont disséqués en retirant des grains les embryons et les pericarpes. Les endospermes sont broyés et 50mg de farine sont utilisés pour l'extraction sélective. Préalablement les α-zéines sont extraites par 3 traitements avec de l'éthanol à 70%. Après centrifugation, l'éthanol est évaporé sous vide. Les protéines insolubles dans l'éthanol (principalement les γ-zéines et les γ-zéines enrichies en lysine) sont extraites du culot avec un tampon de Laemli contenant 10% de mercaptoéthanol (100µl de tampon pour 10mg de farine). Les protéines totales sont ensuite analysées par coloration à l'argent (Morrissey J.H. 1981, Ann. Biochem. vol. 117, p. 307-310). Les γ-zéines et les γ-zéines enrichies en lysine sont analysées par immunoblot en utilisant respectivement les anticorps αG2 (dilution 1/2000) et αPL (dilution 1/500). Un anticorps anti-anticorps de lapin conjugué à la phosphatase alcaline est utilisé comme anticorps secondaire dans l'immunoblot. Les extraits sont dilués pour permettre leur chargement sur SDS-page selon la méthode analytique utilisée.

### Résultats:

### Accumulation de γ-zéines enrichies en lysine dans les plants 20γZ et 45γZ de maïs transgénique;

La figure 13 montre l'immunoblot des extraits de protéines révélées avec l'antisérum αPL (A,B) et les protéines totales après coloration à l'argent (C). Comme on peut le voir dans la fig13A, 6 plants transgéniques 20γZ ont été testés avec l'antisérum αPL et de la γ-zéine enrichie en lysine est exprimée dans les plants transgéniques A1 et D2 (pistes 1 et 6 respectivement). Dans le plant C1 (piste 4), seules des traces de γ-zéine enrichie en lysine sont observées. Lorsque les extraits des 6 plants transgéniques 45γZ sont chargés sur le gel et marqués avec l'antisérum αPL (Fig 13B), on observe une forte réaction avec l'anticorps pour les transformants B1 et C1 (pistes 3 et 4 respectivement).
Il faut noter que la réaction avec l'anticorps dans les extraits des endospermes des plants 45γZ B1 et C1 est plus forte que dans les extraits des plants 20γZ A1 et D2. Ce résultat est confirmé après coloration à l'argent des gels (fig. 13C) où les 2 types de γ-zéine : endogène et enrichie en lysine, sont colorés. La γ-zéine enrichie en lysine a un poids moléculaire apparent de 30kDa et la γ-zéine endogène de 28 kDa. L'expression de γ-zéine enrichie en lysine est plus faible dans le plant 45γZ B1 que dans C1 (pistes 2 et 3 respectivement). Dans les pistes 1 à 6 de la Fig 13C, une dilution identique des protéines des extraits d'endospermes des plants 45γZ et 20γZ avait été déposée sur le gel. A cette dilution, l'expression de la γ-zéine enrichie en lysine est détectée seulement dans les endospermes des plants 45γZ B1 et C1. Cependant, lorsqu'un extrait plus important (40 µg de protéines par piste) de protéines 20γZ est chargé sur le gel, une légère bande (voir flèche), correspondant à la γ-zéine enrichie en lysine est détectée dans les endospermes 20γZ A1 et D2. Ce résultat indique que les plants 45γZ accumulent beaucoup plus de protéines selon l'invention que les plants 20γZ. Ceci est probablement dû aux activités différentes des promoteurs. Les plantes 45γZ ont été transformées avec un construit contenant la γ-zéine selon l'invention sous le contrôle du promoteur γ-zéine (1,7 kb) alors que les plantes 20γZ ont été transformées avec la même séquence codante mais sous le contrôle du promoteur 35S du CaMV. La coloration à l'argent est une technique générale de coloration des protéines, mais la forte couleur marron est surtout observée en présence de protéines basiques. Comme les α-zéines ont été extraites de la farine, comme décrit ci-dessus, elles sont absentes lors de l'analyse en SDS-page de la Fig 13C.

### Ségrégation

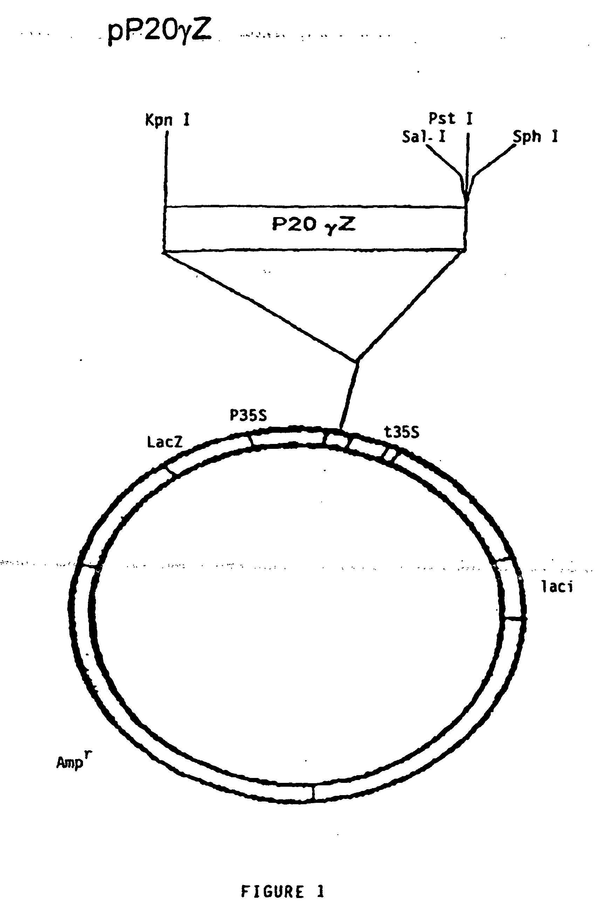

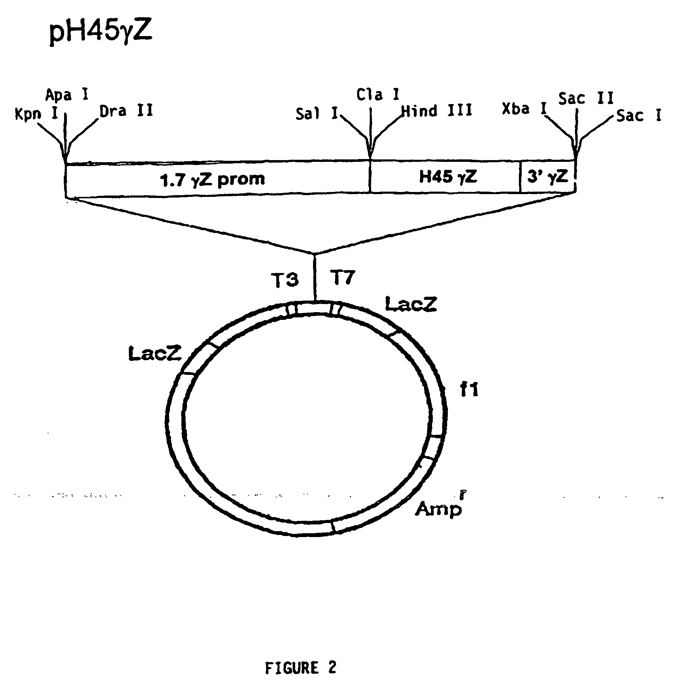

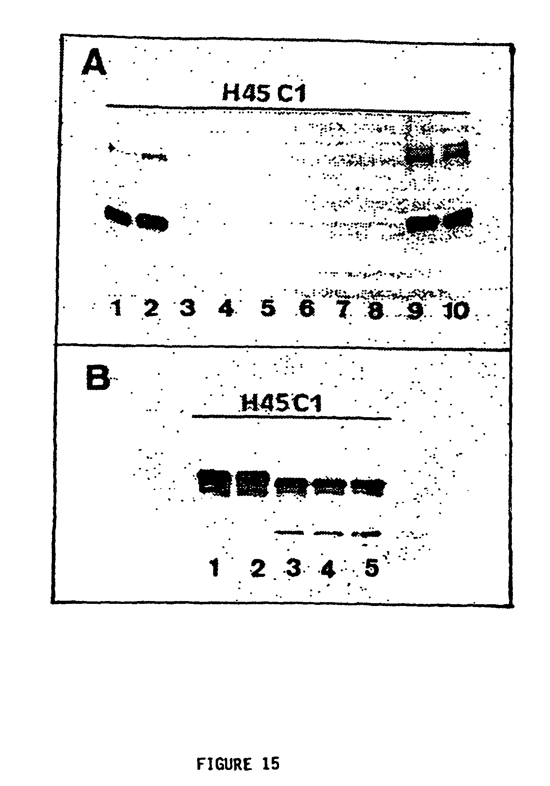

Puisqu'il y a une ségrégation de 50% dans toutes les plantes transgéniques dans le cas d'un locus unique, une analyse grain par grain a été réalisée afin de quantifier le taux de γ-zéine enrichie en lysine dans chaque transformant. L'analyse a été faite uniquement avec les transformants 45γZ qui présentaient le plus fort taux d'expression. La figure 14 montre la coloration à l'argent (A) et l'immunoblot avec αPL (B) de 5 endospermes différents de 45γZ B1 et C1. La faible bande électrophorétique présente sur toutes les pistes (voir A, pistes 1 à 10) correspond à la γ-zéine endogène. Comme on peut le voir dans la Fig 14 A, 2 des 5 endospermes accumulent de la γ-zéine enrichie en lysine (voir pistes 3,4 et 6,7). Par conséquent, environ la moitié des graines accumulent des quantités significatives de protéines enrichies en lysine. Si on prend en compte le fait que des quantités identiques de protéines ont été déposées sur le gel, on observe que le transformant 45γZ C1 a accumulé plus de γ-zéine enrichie en lysine que B1. En fait ce résultat est en accord avec ce qui est observé lors de la coloration à l'argent des extraits mélangés d'endosperme (Fig 13 C, piste 3). La preuve de la présence de γ-zéine enrichie en lysine dans ces endospermes est soulignée dans la Fig 14B. L'immunoblot utilisant l'antisérum αPL montre que 2 extraits d'endosperme de 45γZ B1 (pistes 3 et 4) et de 45γZ C1 (pistes 4 et 6) accumulent de la γ-zéine enrichie en lysine. Pour confirmer le pourcentage de grains transgéniques, 10 nouveaux grains de transformant 45γZ C1 ont été analysés par immunoblot et utilisation de l'antisérum αPL (Fig 15 A). Comme attendu, environ la moitié des graines transgéniques sont détectées. Une bande immunoréactive a été observée dans les extraits d'endosperme (pistes 1,2,9 et 10).

### Estimation de la quantité de γ-zéines enrichies en lysine dans les endospermes des transformants 45γZ;

αG2 est un antisérum polyclonal qui reconnaît les γ-zéines endogènes et les γ-zéines enrichies en lysine. La réactivité de cet antisérum avec les extraits d'endospermes 45γZ C1 a été utilisée pour quantifier la quantité de γ-zéines enrichies en lysine selon l'invention dans les endospermes des plantes transformées.

La figure 15B présente l'immunoblot de 5 extraits protéiques correspondant à 5 endospermes 45γZ C1 (pistes 1 à 5). Comme attendu, seuls 2 endospermes ont présenté un profil d'immunoréaction caractéristique des grains transgéniques. La bande supérieure de 30 kDa correspond à la γ-zéine enrichie en lysine (flèches sur piste 1 et 2) et la bande inférieure à la γ-zéine endogène. On peut noter que dans les endospermes des plantes non transgéniques la bande supérieure est absente (pistes 3, 4 et 5). De façon surprenante, il semble que le contenu en γ-zéine endogène est plus faible dans les extraits des plantes transgéniques que dans les plantes non-transgéniques (voir flèches sur pistes 1 et 5).
A première vue, il a été observé que :
i) dans les endospermes transgéniques 45γZ C1, le ratio γ-zéine enrichie en lysine/γ-zéine endogène est de 7/3. Donc la quantité de protéine modifiée selon l'invention est au moins 2 fois plus importante que celle de la protéine endogène,
ii) la quantité de γ-zéine endogène dans les endospermes non transgéniques (voir pistes 3, 4 et 5) était équivalente à celle de la γ-zéine enrichie en lysine dans les endospermes transgéniques (voir pistes 1 et 2).

### 7) Expression de γ-zéines enrichies en lysine recombinantes dans du blé.

Comme dans l'exemple 6), il est possible de montrer la présence de γ-zéines enrichies en lysine selon l'invention dans du blé.
La transformation du blé peut notamment être effectuée selon la méthode décrite dans Weeks et al., 1993, Plant Physiol., vol 102 : pages 1077-1084 ou selon la méthode décrite dans EP709462.

## Revendications

1. Séquence de nucléotides recombinante comprenant un enchaînement de nucléotides codant pour une protéine de réserve de la famille des zéines, **caractérisée en ce qu'**elle comprend en outre un oligonucléotide choisi parmi :
a) un oligonucléotide comprenant au moins un enchaînement codant pour un polypeptide répondant à la formule (P-K)n, dans laquelle:
- n est un entier supérieur ou égal à 2,
- P représente un résidu d'acide aminé Proline,
- K représente un résidu d'acide aminé Lysine,
le signe "-" symbolise une liaison entre les deux résidus d'acides aminés et notamment une liaison de type peptidique, les n unités (P-K) étant liées entre elles également par de telles liaisons par exemple de type peptidique,
b) un oligonucléotide selon a) dans lequel n est un entier supérieur ou égal à 3, de préférence n est égal à 4, 5, 6, 7, 8, 9,10 ou 15,
c) un oligonucléotide selon a) ou b) dans lequel la séquence des n unités (PK) est interrompue par un ou plusieurs résidus d'acides aminés différents des résidus P ou K,
d) un oligonucléotide selon a), b) ou c) complété à son extrémité 5' et/ou à son extrémité 3' par un ou plusieurs codons,
e) un oligonucléotide selon a), b), c) ou d) complété à son extrémité 5' et/ou à son extrémité 3', par un résidu lysine à l'extrémité N-terminale du polypeptide formé,
f) un oligonucléotide selon e) codant pour un polypeptide répondant à la formule (P-K)n, à la formule K(P-K)₄ ou à la formule 2K(P-K)₄ ;
ledit oligonucléotide étant inséré en un site de l'enchaînement de nucléotides choisi de façon telle que:
- l'expression de la séquence de nucléotides dans une cellule végétale déterminée permet d'obtenir une protéine de réserve de la famille des zéines modifiée localisée de façon identique ou similaire à la protéine de réserve normale qui serait exprimée dans les mêmes conditions dans la même cellule par l'enchaînement de nucléotides codant correspondant, et/ou
- la protéine de réserve de la famille des zéines modifiée codée par la séquence de nucléotides recombinante est reconnue immunologiquement par des anticorps produits contre la protéine de réserve normale correspondante.

2. Séquence de nucléotides recombinante selon la revendication 1, **caractérisée en ce que** l'oligonucléotide est inséré au niveau de l'extrémité N- ou C-terminale de l'enchaînement de nucléotides.

3. Séquence de nucléotides recombinante selon la revendication 1, **caractérisée en ce que** l'oligonucléotide est inséré au niveau de répétitions en tandem riches en proline de l'enchaînement de nudéotides.

4. Séquence de nudéotides selon l'une des revendications 1 à 3, **caractérisée en ce que** l'enchaînement de nucléotides code pour la γ-zéine du maïs.

5. Séquence de nucléotides selon la revendication 4, **caractérisée en ce que** l'enchaînement de nucléotides codant pour la γ-zéine de maïs répond à la séquence présentée sur la Figure 9.

6. Séquence de nucléotides selon la revendication 4 ou 5, **caractérisée en ce que** l'oligonucléotide est inséré à la place ou à la suite du domaine Pro-X ou dans le domaine Pro-X naturellement présent dans la γ-zéine du maïs.

7. Séquence de nucléotides recombinante, **caractérisée en ce qu'**elle comprend une séquence de nucléotides selon l'une quelconque des revendications 1 à 6 sous le contrôle d'un promoteur d'expression.

8. Séquence de nudéotides recombinante selon la revendication 7, **caractérisée en ce que** le promoteur est un promoteur spécifique d'un tissu cellulaire déterminé, par exemple un promoteur spécifique pour l'expression dans les graines, et/ou dans les feuilles de plantes.

9. Séquence de nucléotides selon la revendication 8, **caractérisée en ce que** le promoteur d'expression est celui de la γ-zéine du maïs.

10. Séquence de nucléotides selon la revendication 7 **caractérisée en ce que** le promoteur d'expression est le promoteur CaMV35S.

11. Séquence de nucléotides selon l'une quelconque des revendications 6 à 10, **caractérisée en ce qu'**elle code pour l'un des polypeptides P20yZ ou H45yZ répondant respectivement aux séquences représentées aux figures 11 et 10.

12. Vecteur de clonage et/ou d'expression, **caractérisé en ce qu'**il comprend, en un site non essentiel pour sa réplication, une séquence de nucléotides selon l'une quelconque des revendications 1 à 11.

13. Vecteur de clonage et/ou d'expression, **caractérisé en ce qu'**il s'agit de run des plasmides pP20yZ (CNCMN I-1640) ou pH45yZ (CNCMNI-1639), ou **en ce qu'**il comprend un oligonucléotide codant pour le polypeptide H30yZ représenté à la Figure 3.

14. Polypeptide codé par une séquence selon l'une quelconque des revendications 1 à 6.

15. γ-zéine de maïs modifiée riche en lysine, **caractérisée en ce qu'**elle est codée par la séquence de nucléotides selon la revendication 5.

16. γγ-zéine de maïs modifiée enrichie en lysine, **caractérisée en ce que** sa séquence d'acides aminés est modifiée par au moins un polypeptide répondant à la formule (P-K)n ou à la formule K-(P-K)n, dans laquelle:
- n est un entier supérieur ou égal à 2,
- P représente un résidu d'acide aminé Proline,
- K représente un résidu d'acide aminé Lysine,
- le signe "-" symbolise une liaison entre les deux résidus d'acides aminés et notamment une liaison de type peptidique, les n unités (P-K) étant liées par des liaisons notamment de type peptidique,
ledit polypeptide répondant à la formule(P-K)n ou K-(P-K)n étant inséré dans la séquence d'acides aminés de la γ-zéine de maïs normale de telle façon que:
- lorsque la γ-zéine modifiée riche en lysine est produite dans une cellule hôte, notamment une cellule végétale, elle est localisée de façon identique ou similaire à la γ-zéine de maïs normale qui serait produite dans les mêmes conditions, dans la même cellule hôte, et/ou,
- la γ-zéine de maïs modifiée est reconnue par des anticorps dirigés contre la γ-zéine normale de maïs.

17. γ-zéine de maïs modifiée enrichie en lysine, selon la revendication 16 **caractérisée en ce que** ledit polypeptide est substitué à une séquence de répétitions en tandem riches en Proline naturellement présente dans la γ-zéine de maïs normale.

18. γ-zéine de maïs modifiée enrichie en lysine, selon la revendication 16 **caractérisée en ce que** ledit polypeptide est inséré avec délétion d'un ou plusieurs acides aminés d'une séquence de répétitions en tandem riches en Proline de la γ-zéine normale.

19. γ-zéine de maïs modifiée enrichie en lysine, selon la revendication 16 **caractérisée en ce que** ledit polypeptide est ajouté dans une séquence de répétitions en tandem riches en Proline de la γ-zéine normale.

20. γ-zéine de maïs modifiée enrichie en lysine, selon la revendication 16, **caractérisée en ce que** ledit polypeptide est inséré dans la partie N- ou C-terminale de la séquence d'acides aminés de la γ-zéine de maïs normale.

21. γ-zéine de maïs modifiée selon la revendication 14, **caractérisée en ce qu'**il s'agit de la protéine P20yZ, figure 11, ou de la protéine H30yZ, figure 3, ou de la protéine H45yZ, figure 10.

22. Cellule hôte recombinante, **caractérisée en ce qu'**elle comprend une séquence de nucléotides selon l'une quelconque des revendications 1 à 11.

23. Cellule hôte selon la revendication 22, **caractérisée en ce qu'**il s'agit d'une bactérie, par exemple de *E*. *coli* ou *Agrobacterium tumefaciens.*

24. Cellule hôte selon la revendication 22, **caractérisée en ce qu'**il s'agit d'une cellule végétale.

25. Cellule hôte selon la revendication 24, **caractérisée en ce qu'**il s'agit d'une cellule de semences de plante.

26. Cellule hôte selon la revendication 25, **caractérisée en ce qu'**il s'agit d'une cellule d'endosperme de semences de maïs.

27. Cellule hôte selon la revendication 26, **caractérisée en ce qu'**elle contient, intégrée de façon stable dans son génome, une séquence de nucléotides selon la revendication 5.

28. Cellule hôte selon la revendication 26, **caractérisée en ce qu'**elle produit une γ-zéine de maïs modifiée riche en lysine selon l'une des revendications 16 to 21.

29. Cellule hôte selon la revendication 24, **caractérisée en ce qu'**il s'agit d'une cellule de soja, de tournesol de tabac, de blé, d'avoine, de luzerne, de riz, de colza, d'*Arabidopsis* ou de maïs.

30. Semences produisant un polypeptide selon l'une quelconque des revendications 16 à 21.

31. Plante produisant un polypeptide selon l'une quelconque des revendications 16 à 21.

32. Plante selon la revendication 31, **caractérisée en ce qu'**il s'agit du maïs.

33. Semences exprimant un polypeptide modifié, obtenues à partir des plantes selon la revendication 31 ou 32.

34. Utilisation d'un oligonucléotide choisi parmi :
a) un oligonucléotide comprenant au moins un enchaînement codant pour un polypeptide répondant à la formule (P-K)n, dans laquelle:
- n est un entier supérieur ou égal à 2,
- P représente un résidu d'acide aminé Proline,
- K représente un résidu d'acide aminé Lysine,
le signe "-" symbolise une liaison entre les deux résidus d'acides aminés et notamment une liaison de type peptidique, les n unités (P-K) étant liées entre elles également par de telles liaisons par exemple de type peptidique,
b) un oligonucléotide selon a) dans lequel n est un entier supérieur ou égal à 3, de préférence n est égal à 4, 5, 6, 7, 8, 9,10 ou 15,
c) un oligonucléotide selon a) ou b) dans lequel la séquence des n unités (PK) est interrompue par un ou plusieurs résidus d'acides aminés différents des résidus P ou K,
d) un oligonucléotide selon a), b) ou c) complété à son extrémité 5' et/ou à son extrémité 3' par un ou plusieurs codons,
e) un oligonucléotide selon a), b), c) ou d) complété à son extrémité 5' et/ou à son extrémité 3', par un résidu lysine à l'extrémité N-terminale du polypeptide formé,
f) un oligonucléotide selon e) codant pour un polypeptide répondant à la formule (P-K)n, à la formule K(P-K)₄ ou à la formule 2K(P-K)₄
pour l'obtention d'une protéine de réserve de la famille des zéines enrichie en Lysine.

35. Procédé de production de plantes ou de semences exprimant une protéine de réserve de la famille des zéines modifiée, **caractérisé en ce qu'**il comprend les étapes de:
a) transformation d'une cellule végétale, avec une séquence de nucléotides selon l'une quelconque des revendications 1 à 11, ou un vecteur selon l'une quelconque des revendications 12 ou 13, dans des conditions permettant l'expression de façon stable et fonctionnelle de la protéine de réserve modifiée codée par la séquence de nucléotides;
b) régénération de plantes à partir de la cellule de plante transformée de l'étape a), pour obtenir des plantes exprimant la protéine de réserve modifiée,
c) le cas échéant, obtention de semences à partir des plantes modifiées obtenues à l'étape b).

36. Procédé selon la revendication 35, **caractérisé en ce que** la plante est le maïs, et la protéine de réserve est la γ-zéine.

## Patentansprüche

1. Rekombinante Nukleotidsequenz, die eine Verknüpfung von Nukleotiden, die für ein Speicherprotein der Familie der Zeine codieren, umfaßt, **dadurch gekennzeichnet, daß** sie weiterhin ein Oligonukleotid umfaßt, das aus der Reihe:
a) Oligonukleotid, das mindestens eine Verknüpfung, die für ein Polypeptid der Formel (P-K)n, in der:
- n eine ganze Zahl 2 oder darüber bedeutet,
- P einen Prolin-Aminosäurerest darstellt,
- K einen Lysin-Aminosäurerest darstellt,
das Zeichen "-" eine Bindung zwischen den beiden Aminosäureresten, insbesondere eine peptidartige Bindung, symbolisiert, wobei die n Einheiten (P-K) untereinander ebenfalls durch solche Bindungen, zum Beispiel peptidartige Bindungen, gebunden sind,
codiert, umfaßt,
b) Oligonukleotid gemäß a), in dem n eine ganze Zahl 3 oder darüber, vorzugsweise 4, 5, 6, 7, 8, 9, 10 oder 15, bedeutet,
c) Oligonukleotid gemäß a) oder b), in dem die Sequenz der n Einheiten (PK) durch einen oder mehrere Aminosäurereste, die von den Resten P oder K verschieden sind, unterbrochen ist,
d) Oligonukleotid gemäß a), b) oder c), das an seinem 5'- und/oder 3'-Ende durch ein oder mehrere Codons vervollständigt ist,
e) Oligonukleotid gemäß a), b), c) oder d), das an seinem 5'- und/oder 3'-Ende durch einen Lysinrest am N-terminalen Ende des gebildeten Polypeptids vervollständigt ist,
f) Oligonukleotid gemäß e), das für ein Polypeptid der Formel (P-K)n, der Formel K(P-K)₄ oder der Formel 2K(P-K)₄ codiert,
stammt,
wobei das Oligonukleotid an einer Stelle der Nukleotidverknüpfung insertiert ist, die so gewählt ist, daß
- man durch die Expression der Nukleotidsequenz in einer bestimmten Pflanzenzelle ein modifiziertes Speicherprotein der Familie der Zeine erhält, das gleich oder ähnlich wie das normale Speicherprotein, das unter den gleichen Bedingungen in der gleichen Zelle durch die entsprechende codierende Nukleotidverknüpfung exprimiert würde, lokalisiert ist, und/oder
- das modifizierte Speicherprotein der Familie der Zeine, das von der rekombinanten Nukleotidsequenz codiert wird, von gegen das entsprechende normale Speicherprotein erzeugten Antikörpern immunologisch erkannt wird.

2. Rekombinante Nukleotidsequenz nach Anspruch 1, **dadurch gekennzeichnet, daß** das Oligonukleotid am N- oder C-terminalen Ende der Nukleotidverknüpfung insertiert ist.

3. Rekombinante Nukleotidsequenz nach Anspruch 1, **dadurch gekennzeichnet, daß** das Oligonukleotid an den prolinreichen in Tandemanordnung wiederholten Sequenzen der Nukleotidverknüpfung insertiert ist.

4. Nukleotidsequenz nach einem der Ansprüche 1 bis 3, **dadurch gekennzeichnet, daß** die Nukleotidverknüpfung für das Mais-γ-Zein codiert.

5. Nukleotidsequenz nach Anspruch 4, **dadurch gekennzeichnet, daß** die für das Mais-γ-Zein codierende Nukleotidverknüpfung der in Abbildung 9 dargestellten Sequenz entspricht.

6. Nukleotidsequenz nach Anspruch 4 oder 5, **dadurch gekennzeichnet, daß** das Oligonukleotid an der Stelle oder nach der Pro-X-Domäne oder in der Pro-X-Domäne, die im Mais-γ-Zein natürlich vorkommt, insertiert ist.

7. Rekombinante Nukleotidsequenz, **dadurch gekennzeichnet, daß** sie eine Nukleotidsequenz nach einem der Ansprüche 1 bis 6 unter der Kontrolle eines Expressionspromoters umfaßt.

8. Rekombinante Nukleotidsequenz nach Anspruch 7, **dadurch gekennzeichnet, daß** sie sich bei dem Promoter um einen für ein bestimmtes Zellgewebe spezifischen Promoter, zum Beispiel um einen für die Expression in den Samenkörnern und/oder in den Blättern von Pflanzen spezifischen Promoter, handelt.

9. Nukleotidsequenz nach Anspruch 8, **dadurch gekennzeichnet, daß** es sich bei dem Expressionspromoter um den des Mais-γ-Zeins handelt.

10. Nukleotidsequenz nach Anspruch 7, **dadurch gekennzeichnet, daß** es sich bei dem Expressionspromoter um den CaMV35S-Promoter handelt.

11. Nukleotidsequenz nach einem der Ansprüche 6 bis 10, **dadurch gekennzeichnet, daß** sie für eines der Polypeptide P20yZ oder H45yZ, die den in Abbildung 11 bzw. 10 beschriebenen Sequenzen entsprechen, codiert.

12. Klonierungs- und/oder Expressionsvektor, **dadurch gekennzeichnet, daß** er an einer für seine Replikation nicht unbedingt erforderlichen Stelle eine Nukleotidsequenz nach einem der Ansprüche 1 bis 11 umfaßt.

13. Klonierungs- und/oder Expressionsvektor, **dadurch gekennzeichnet, daß** es sich um eines der Plasmide pP20yZ (CNCMN I-1640) oder pH45yZ (CNCMN 1-1639) handelt, oder **dadurch**, daß er ein für das in Abbildung 3 dargestellte Polypeptid H30yZ codierendes Oligonukleotid, umfaßt.

14. Polypeptid, das von einer Sequenz nach einem der Ansprüche 1 bis 6 codiert wird.

15. An Lysin angereichertes modifiziertes Mais-γ-Zein, **dadurch gekennzeichnet, daß** es von der Nukleotidsequenz nach Anspruch 5 codiert wird.

16. An Lysin angereichertes modifiziertes Mais-γ-Zein, **dadurch gekennzeichnet, daß** seine Aminosäuresequenz mit mindestens einem Polypeptid der Formel (P-K)n oder der Formel K-(P-K)n, in der:
- n eine ganze Zahl 2 oder darüber bedeutet,
- P einen Prolin-Aminosäurerest darstellt,
- K einen Lysin-Aminosäurerest darstellt,
- das Zeichen "-" eine Bindung zwischen den beiden Aminosäureresten, insbesondere eine peptidartige Bindung, symbolisiert, wobei die n Einheiten (P-K) untereinander ebenfalls durch solche Bindungen, zum Beispiel peptidartige Bindungen, gebunden sind,
modifiziert ist,
wobei das Polypeptid der Formel (P-K)n oder K(P-K)n so in die Aminosäuresequenz des normalen Mais-γ-Zein insertiert ist, daß
- wenn das lysinreiche modifizierte γ-Zein in einer Wirtszelle, insbesondere einer Pflanzenzelle produziert wird, es auf gleiche oder ähnliche Weise wie das normale Mais-γ-Zein, das unter den gleichen Bedingungen in der gleichen Wirtszelle produziert würde, lokalisiert ist, und/oder
- das modifizierte Mais-γ-Zein von gegen das normale Mais-γ-Zein gerichteten Antikörpern erkannt wird.

17. An Lysin angereichertes modifiziertes Mais-γ-Zein nach Anspruch 16, **dadurch gekennzeichnet, daß** durch dieses Polypeptid eine in normalem Mais-γ-Zein natürlich vorhandene prolinreiche in Tandemanordnung wiederholte Sequenz ersetzt wird.

18. An Lysin angereichertes modifiziertes Mais-γ-Zein nach Anspruch 16, **dadurch gekennzeichnet, daß** das Polypeptid unter Deletion von einer oder mehreren Aminosäuren einer prolinreichen in Tandemanordnung wiederholten Sequenz des normalen γ-Zeins insertiert ist.

19. An Lysin angereichertes modifiziertes Mais-γ-Zein nach Anspruch 16, **dadurch gekennzeichnet, daß** das Polypeptid einer prolinreichen in Tandemanordnung wiederholten Sequenz des normalen γ-Zeins hinzugefügt ist.

20. An Lysin angereichertes modifiziertes Mais-γ-Zein nach Anspruch 16, **dadurch gekennzeichnet, daß** das Polypeptid in den N- oder C-terminalen Teil der Aminosäuresequenz des normalen Mais-γ-Zeins insertiert ist.

21. Modifiziertes Mais-γ-Zein nach Anspruch 14, **dadurch gekennzeichnet, daß** es sich um das Protein P20yZ, Abbildung 11, oder das Protein H30yZ, Abbildung 3, oder das Protein H45yZ, Abbildung 10, handelt.

22. Rekombinante Wirtszelle, **dadurch gekennzeichnet, daß** sie eine Nukleotidsequenz nach einem der Ansprüche 1 bis 11 umfaßt.

23. Wirtszelle nach Anspruch 22, **dadurch gekennzeichnet, daß** es sich um ein Bakterium, z.B. um *E. coli* oder *Agrobacterium tumefaciens,* handelt.

24. Wirtszelle nach Anspruch 22, **dadurch gekennzeichnet, daß** es sich um eine pflanzliche Zelle handelt.

25. Wirtszelle nach Anspruch 24, **dadurch gekennzeichnet, daß** es sich um eine Pflanzensamenzelle handelt.

26. Wirtszelle nach Anspruch 25, **dadurch gekennzeichnet, daß** es sich um eine Maissamen-Endospermzelle handelt.

27. Wirtszelle nach Anspruch 26, **dadurch gekennzeichnet, daß** sie eine Nukleotidsequenz nach Anspruch 5 stabil in ihr Genom integriert enthält.

28. Wirtszelle nach Anspruch 26, **dadurch gekennzeichnet, daß** sie ein lysinreiches modifiziertes Mais-γ-Zein nach einem der Ansprüche 16 bis 21 produziert.

29. Wirtszelle nach Anspruch 24, **dadurch gekennzeichnet, daß** es sich um eine Soja-, Sonnenblumen-, Tabak-, Weizen-, Hafer-, Luzerne-, Reis-, Raps-, *Arabidopsis-*oder Maiszelle handelt.

30. Samen, die ein Polypeptid nach einem der Ansprüche 16 bis 21 produzieren.

31. Pflanze, die ein Polypeptid nach einem der Ansprüche 16 bis 21 produziert.

32. Pflanze nach Anspruch 31, **dadurch gekennzeichnet, daß** es sich um Mais handelt.

33. Samen, die ein modifiziertes Polypeptid exprimieren und die von Pflanzen nach Anspruch 31 oder 32 erhalten werden.

34. Verwendung eines Oligonukleotids, das aus der Reihe:
a) Oligonukleotid, das mindestens eine Verknüpfung, die für ein Polypeptid der Formel (P-K)n, in der:
- n eine ganze Zahl 2 oder darüber bedeutet,
- P einen Prolin-Aminosäurerest darstellt,
- K einen Lysin-Aminosäurerest darstellt,
das Zeichen "-" eine Bindung zwischen den beiden Aminosäureresten, insbesondere eine peptidartige Bindung, symbolisiert, wobei die n Einheiten (P-K) untereinander ebenfalls durch solche Bindungen, zum Beispiel peptidartige Bindungen, gebunden sind,
codiert, umfaßt,
b) Oligonukleotid gemäß a), in dem n eine ganze Zahl 3 oder darüber, vorzugsweise 4, 5, 6, 7, 8, 9, 10 oder 15, bedeutet,
c) Oligonukleotid gemäß a) oder b), in dem die Sequenz der n Einheiten (PK) durch einen oder mehrere Aminosäurereste, die von den Resten P oder K verschieden sind, unterbrochen ist,
d) Oligonukleotid gemäß a), b) oder c), das an seinem 5'- und/oder 3'-Ende durch ein oder mehrere Codons vervollständigt ist,
e) Oligonukleotid gemäß a), b), c) oder d), das an seinem 5'- und/oder 3'-Ende durch einen Lysinrest am N-terminalen Ende des gebildeten Polypeptids vervollständig ist,
f) Oligonukleotid gemäß e), das für ein Polypeptid der Formel (P-K)n, der Formel K(P-K)₄ oder der Formel 2K(P-K)₄ codiert,
stammt,
zur Gewinnung eines an Lysin angereicherten Speicherproteins aus der Familie der Zeine.

35. Verfahren zur Herstellung von Pflanzen oder Samen, die ein modifiziertes Speicherprotein aus der Familie der Zeine exprimieren, **dadurch gekennzeichnet, daß** es die folgenden Schritte umfaßt:
a) Transformation einer Pflanzenzelle mit einer Nukleotidsequenz nach einem der Ansprüche 1 bis 11 oder einem Vektor nach einem der Ansprüche 12 oder 13 unter Bedingungen, die die stabile und funktionelle Expression des von der Nukleotidsequenz codierten modifizierten Speicherproteins gestatten;
b) Regeneration von Pflanzen aus der transformierten Pflanzenzelle aus Schritt a) zur Gewinnung von Pflanzen, die das modifizierte Speicherprotein exprimieren, sowie
c) gegebenenfalls Gewinnung von Samen von in Schritt b) gewonnenen modifizierten Pflanzen.

36. Verfahren nach Anspruch 35, **dadurch gekennzeichnet, daß** es sich bei der Pflanze um Mais und bei dem Speicherprotein um γ-Zein handelt.

## Claims

1. Recombinant nucleotide sequence comprising a series of nucleotides coding for a storage protein from the zein family, **characterized in that** it furthermore comprises an oligonucleotide selected from amongst:
a) an oligonucleotide comprising at least one series coding for a polypeptide of the formula (P-K)n in which:
- n is an integer of 2 or above,
- P represents a proline amino acid residue,
- K represents a lysine amino acid residue,
the symbol "-" denotes a bond between the two amino acid residues, in particular a peptide-type bond, the n units (P-K) likewise being linked to each other by such bonds, for example peptide-type bonds,
b) an oligonucleotide as described in a) in which n is an integer of 3 or above, preferably n is 4, 5, 6, 7, 8, 9, 10 or 15,
c) an oligonucleotide as described in a) or b) in which the sequence of the n units (PK) is interrupted by one or more amino acid residues which differ from the residues P or K,
d) an oligonucleotide as described in a), b) or c) which is completed at its 5' and/or 3' end by one or more codons,
e) an oligonucleotide as described in a), b), c) or d) which is completed at its 5' and/or 3' end by a lysine residue at the N-terminal end of the polypeptide formed,
f) an oligonucleotide as described in e) which codes for a polypeptide of the formula (P-K)n, the formula K(P-K)₄ or the formula 2K(P-K)₄;
said oligonucleotide being inserted at a site of the nucleotide series which is chosen in such a way that
- the expression of the nucleotide sequence in a particular plant cell allows a modified storage protein of the zein family to be obtained whose localization is identical or similar to the normal storage protein which would be expressed by the corresponding coding nucleotide series under the same conditions in the same cell, and/or
- the modified storage protein of the zein family which is encoded by the recombinant nucleotide sequence is recognized immunologically by antibodies raised against the corresponding normal storage protein.

2. Recombinant nucleotide sequence according to Claim 1, **characterized in that** the oligonucleotide is inserted at the N- or C-terminal end of the nucleotide series.

3. Recombinant nucleotide sequence according to Claim 1, **characterized in that** the oligonucleotide is inserted at proline-rich tandem repeats of the nucleotide series.

4. Nucleotide sequence according to one of Claims 1 to 3, **characterized in that** the nucleotide series codes for maize γ-zein.

5. Nucleotide sequence according to Claim 4, **characterized in that** the nucleotide series which codes for maize γ-zein has the sequence shown in Figure 9.

6. Nucleotide sequence according to Claim 4 or 5, **characterized in that** the oligonucleotide is inserted instead of or following the pro-X domain or in the pro-X domain which is naturally present in maize γ-zein.

7. Recombinant nucleotide sequence, **characterized in that** it comprises a nucleotide sequence according to any of Claims 1 to 6 under the control of an expression promoter.

8. Recombinant nucleotide sequence according to Claim 7, **characterized in that** the promoter is a promoter which is specific for a particular cell tissue, for example a promoter which is specific for expression in the seeds and/or the leaves of plants.

9. Nucleotide sequence according to Claim 8, **characterized in that** the expression promoter is the promoter of maize γ-zein.

10. Nucleotide sequence according to Claim 7, **characterized in that** the expression promoter is the CaMV35S promoter.

11. Nucleotide sequence according to any of Claims 6 to 10, **characterized in that** it codes for one of the polypeptides P20yZ or H45yZ, which have the sequences shown in Figures 11 and 10, respectively.

12. Cloning and/or expression vector, **characterized in that** it comprises a nucleotide sequence according to any of Claims 1 to 11 at a site which is not essential for its replication.

13. Cloning and/or expression vector, **characterized in that** it is one of the plasmids pP20yZ (CNCMN I-1640) or pH45yZ (CNCMN I-1639), or **in that** it comprises an oligonucleotide coding for the polypeptide H30yZ, which is shown in Figure 3.

14. Polypeptide encoded by a sequence according to any of Claims 1 to 6.

15. Lysine-rich modified maize γ-zein, **characterized in that** it is encoded by the nucleotide sequence according to Claim 5.

16. Lysine-enriched modified maize γ-zein, **characterized in that** its amino acid sequence is modified by at least one polypeptide of the formula (P-K)n or the formula K-(P-K)n in which:
- n is an integer of 2 or above,
- P represents a proline amino acid residue,
- K represents a lysine amino acid residue,
- the symbol "-" denotes a bond between the two amino acid residues, in particular a peptide-type bond, the n units (P-K) being linked by bonds, in particular peptide-type bonds, said polypeptide which has the formula (P-K)n or K-(P-K)n being inserted in the normal maize γ-zein amino acid sequence in such a way that
- when the lysine-rich modified γ-zein is produced in a host cell, in particular a plant cell, its localization is identical or similar to normal maize γ-zein which would be produced under the same conditions in the same host cell, and/or
- the modified maize γ-zein is recognized by antibodies directed against normal maize γ-zein.

17. Lysine-enriched modified maize γ-zein according to Claim 16, **characterized in that** said polypeptide is substituted for a proline-rich tandem repeat sequence which is naturally present in normal maize γ-zein.

18. Lysine-enriched modified maize γ-zein according to Claim 16, **characterized in that** said polypeptide is inserted with deletion of one or more amino acids of a proline-rich tandem repeat sequence of normal γ-zein.

19. Lysine-enriched modified maize γ-zein according to Claim 16, **characterized in that** said polypeptide is added into a proline-rich tandem repeat sequence of normal γ-zein.

20. Lysine-enriched modified maize γ-zein according to Claim 16, **characterized in that** said polypeptide is inserted in the N- or C-terminal part of the amino acid sequence of normal maize γ-zein.

21. Modified maize γ-zein according to Claim 14, **characterized in that** it is the protein P20yZ, Figure 11, or the protein H30yZ, Figure 3, or the protein H45yZ, Figure 10.

22. Recombinant host cell, **characterized in that** it comprises a nucleotide sequence according to any of Claims 1 to 11.

23. Host cell according to Claim 22, **characterized in that** it is a bacterium, for example *E. coli* or *Agrobacterium tumefaciens.*

24. Host cell according to Claim 22, **characterized in that** it is a plant cell.

25. Host cell according to Claim 24, **characterized in that** it is a plant seed cell.

26. Host cell according to Claim 25, **characterized in that** it is an endosperm cell or maize seed.

27. Host cell according to Claim 26, **characterized in that** it contains a nucleotide sequence according to Claim 5 stably integrated into its genome.

28. Host cell according to Claim 26, **characterized in that** it produces a lysine-rich modified maize γ-zein according to one of Claims 16 to 21.

29. Host cell according to Claim 24, **characterized in that** it is a soya, sunflower, tobacco, wheat, oat, lucerne, rice, oil seed rape, *Arabidopsis* or maize cell.

30. Seed producing a polypeptide according to any of Claims 16 to 21.

31. Plant producing a polypeptide according to any of Claims 16 to 21.

32. Plant according to Claim 31, **characterized in that** it is maize.

33. Seed expressing a modified polypeptide and obtained from plants according to Claim 31 or 32.

34. Use of an oligonucleotide selected from amongst:
a) an oligonucleotide comprising at least one series coding for a polypeptide of the formula (P-K)n in which:
- n is an integer of 2 or above,
- P represents a proline amino acid residue,
- K represents a lysine amino acid residue,
the symbol "-" denotes a bond between the two amino acid residues, in particular a peptide-type bond, the n units (P-K) likewise being linked to each other by such bonds, for example peptide-type bonds,
b) an oligonucleotide as described in a) in which n is an integer of 3 or above, preferably n is 4, 5, 6, 7, 8, 9, 10 or 15,
c) an oligonucleotide as described in a) or b) in which the sequence of the n units (PK) is interrupted by one or more amino acid residues which differ from the residues P or K,
d) an oligonucleotide as described in a), b) or c) which is completed at its 5' and/or 3' end by one or more codons,
e) an oligonucleotide as described in a), b), c) or d) which is completed at its 5' and/or 3' end by a lysine residue at the N-terminal end of the polypeptide formed,
f) an oligonucleotide as described in e) which codes for a polypeptide of the formula (P-K)n, the formula K(P-K)₄ or the formula 2K(P-K)₄ for obtaining a lysine-enriched storage protein of the zein family.

35. Method of producing plants or seed expressing a modified storage protein of the zein family, **characterized in that** it comprises the following steps:
a) transformation of the plant cell with a nucleotide sequence according to any of Claims 1 to 11, or with a vector according to either of Claims 12 and 13 under conditions which allow the stable and functional expression of the modified storage protein encoded by the nucleotide sequence;
b) regeneration of plants from the transformed plant cell of step a) for obtaining plants expressing the modified storage protein;
c) if appropriate, obtaining seed from modified plants obtained in step b).

36. Method according to Claim 35, **characterized in that** the plant is maize and the storage protein is γ-zein.
